# EUROPEAN PATENT APPLICATION

(11) **EP 1 913 870 A1**
(43) Date of publication of application: **23.04.2008**
(21) Application number: 06425723.1
(22) Date of filing: 19.10.2006
(51) Int. Cl.: A61B 5/055, G06T 7/00, G06F 19/00

(54) **Apparatus for determining indications helping the diagnosis of rheumatic diseases and its method**

(71) Applicant: Esaote S.p.A., 20100 Milano (IT)
(72) Inventor: Biglieri, Eugenio, 15024 Masio (AL) (IT); Satragno, Luigi, 16122 Genova (IT)
(74) Representative: Karaghiosoff, Giorgio Alessandro

(57) **Abstract**

Apparatus for determining indications helping the diagnosis of rheumatic diseases comprising at least a section for detecting and acquiring signals from a body under examination or a part thereof wherein the detection section is a unit for detecting images by nuclear magnetic resonance. In said apparatus there is further integrated a section for processing acquired images as regards image data and/or resonance signals, which processing section from image data and/or resonance signals, determines values of one or more different numerical parameters indicating the presence or absence of a rheumatic disease and/or a measure of the evolution condition of said rheumatic disease.

## Description

The present invention relates to an apparatus for determining indications helping the diagnosis of rheumatic diseases comprising at least a section for detecting and acquiring signals from a body under examination or a part thereof.

Rheumatic diseases are continuously increasing as regards the effect on the world population and they are one of the fields in which medical methods have most developped. Suffice it to mention the announcement of the initiative "Bone and Joint Decade: 2000-2010" made by the World Health Organization having the aim of improving the quality of life (from a medical point of view) of patients suffering from diseases regarding the muscle-skeletal field at a world level. Such diseases are one of the most common reason of invalidity and involve very high social and medical costs (the estimated value is about 215 billion dollars a year in the United States). Particularly rheumatic diseases affect about 10% of population if they are considered as a whole, including also degenerative diseases (so called arthroses). Among these diseases the rheumatoid arthritis affects abouts 1% of the population of developed countries. Practically 90% of people sooner or later will suffer from painful diseases (the well-known backache) being the most important cause limiting working capacities of middle-aged people and one of the greatest cause for requiring medical examinations and generally health controls.

In these fields the magnetic resonance is the "gold standard" diagnostic indagation method or potentially it is such a method.

So called dedicated MRI systems known at present allow the diffusion of the resonance in studying rheumatic diseases that, up to now, have been diagnosed mainly on the basis of clinical data. At present the diagnostic method that is used is the conventional radiology the total inadequacy of which has been shown in finding the disease in precocious stages that at present can be clinically treated by finding specific pharmacologic treatments, whose usefulness increases if they are begun upon the onset of the disease. The diagnosis by conventional radiology is based on highlithing bony erosions that are the effect of the disease after months or years of activity and represent the characteristic indication of a damage that is already occurred.

It is known that it is possible to have information about the potential presence of a disease in its precocious stages and so before the onset of the bony damage by examining the condition of the synovia and its possible inflammation and the presence of possible damages to the cartilage. To this aim the nuclear magnetic resonance examination is an ideal method just for the ability of highlithing synovial inflammatory conditions and possible cartilaginoid damages, obviously besides allowing the detection of bony damages, all that with a panoramic view and a detail that are definitely higher than x-rays.

Despite these intrinsic abilities of the MRI diagnostic imaging and advantages deriving from using MRI particularly dedicated systems for their optimal cost-advantage ratio and the easy in installing them, that can allow to place them at peripherical clinical institutes for the benefit of patients, at present the MRI imaging method is still used in a traditional way, i.e. images are read and analysed from a mere morphologic point of view like radiologic images, therefore above advantages of MRI method are not fully used.

At present many so called CAD systems (computer aided diagnosis) are known which systems work on the basis of different methods for processing image data that are limited in processing diagnostic images, that is image data, in order to recognize from images shapes and objects shown in images or predetermined qualities or kinds of shown objects such as for example healthy tissues or tissues suffering from diseases or lesions. In order to obtain required information or at least predictive or classificating indications or possible morphologic quantities or physical parameters from image data, currently known CAD systems use different kinds of known algorithms. However algorithms are relatively complex to be applied and images are generally analysed off-line by a specialized institute that just receives image data files and processes them then giving desired output data.

Apart from the lack of a direct collaboration between the radiologist i.e. between personnel assigned to acquire images, the physician evaluating images and defining the diagnosis and personnel specialized in processing images for obtaining additional information from the CAD system, there is no direct interaction between the apparatus acquiring images and the CAD processing system. Moreover the centralized off-line system often does not have at disposal a complete database of data about the individual patient which data can be of primary importance both for acquiring subsequent diagnostic images and for processing them since they certainly help in informing or adjusting processing systems and i.e. algorithms aimingly requiring a learning or configuration about details of the patient history. A further drawback is the fact that results from the analysis of the off-line CAD system cannot be used immediately or withing a time length consistent with the length of the examination therefore the patient must return or repeat the examination consequently having an increase in examination costs. Finally but not of minor importance there is the fact that a centralized processing system with CAD functionalities is often redundant and it is not specialized in aimingly analysing or processing diagnostic images for the specific disease therefore for specific information searched in images.

Moreover CAD systems use different kinds of mathematical tools that are used both individually and in combination one with the other and in order to be effective these tools require a coding of morphologic and/or functional conditions by means of numerical parameters. Up to now MRI images have been always read on the basis of a traditional, radiologic method that is merely a visual one and it did not consider possible parameters coding and/or objectively indicating conditions as regards the presence of rheumatic diseases and their evolution degree, so at present these methods cannot be used.

The aim of the present invention is to provide an apparatus for determining indications helping the diagnosis of rheumatic diseases by acquiring MRI images of the type described hereinbefore and by means of relatively simple arrangements allows to provide the radiologist and the doctor with an instrument supporting the diagnosis and quantifying, however possible it is, the possible probable presence or absence and the evolution degree of the pathologic condition considering not only a morphologic reading of image data but also functional characteristics.

Moreover a further aim to be simultaneously achieved is the fact of providing the possibility of a direct synergic interaction between means treating and processing image data for determining indications helping the diagnosis and means acquiring image data such to obtain a synergic optimization of all parameters or acquiring settings in order to achieve the best final result.

The invention achieves the above aims by providing an apparatus for determining indications helping the diagnosis of rheumatic diseases comprising a section detecting and acquiring signals from a body under examination or a part thereof and moreover it is characterized in that the detecting section is a unit detecting images by nuclear magnetic resonance and in said apparatus there is further integrated a section processing acquired images as regards image data and/or resonance signals, which processing section from image data and/or resonance signals defines values of one or more different numerical parameters revealing the presence or absence of a rheumatic disease and/or a measure of the evolution condition of said rheumatic disease.

Therefore in the above apparatus there are integrated both functionalities about the mere acquisition, generation, storage and displaying of MRI images typical of current MRI apparati and functionalities about the processing of image data typical of image processing systems having CAD functionalities (Computer Aided Diagnosis), such as the automatic or semi-automatic recognition of objects represented in images, the automatic or semi-automatic recognition of qualitative, quantitative and/or morphologic and/or dynamic characteristics of objects represented in MRI images and/or possible classifications or predictions of said objects represented by specific areas or volumes of an image with respect to predetermined characteristics, such as also the fact of taking out values of physical or physiologic parameters, whose processing functionalities are carried out in the "on-line" mode by the scan, i.e. immediately after having acquired image data.

As regards the present invention its characteristcs are applied both to two-dimensional images and to three-dimensional images and so the general term image or image data means both two-dimensional images i.e. acquired along one or more section planes, and three-dimensional images i.e. regarding a certain volume of the body under examination.

As regards the device, the integration is relatively simple, since means intended for providing CAD functionalities are composed of so called software "tool", i.e. programs treating data that can be loaded in memories of a computer and executed by it, for example a computer of the personal computer type or the like. It is to be noted that even a part of processes generating and displaying images that conventionally are carried out by the apparatus acquiring MRI images can be composed of softwares that are executed by computers and that typically tomographs acquring MRI images comprise computers in the form of personal computers or computers with dedicated hardware able to execute another code. Therefore the integration of functionalities of CAD systems in the tomograph from the constructive point of view requires at most an upgrade of mass and buffer memories and/or of the computation skill, i.e. of the processor and of elements cooperating with it, as well as the development of CAD processing software.

By integrating means acquiring, generating and displaying MRI images with image processing means having CAD functionalities or of the type defined above, the invention allows to easily expand the kind of data to be processed.

According to an advantageous embodiment data acquired by the tomograph and subjected to the analysis by processing means having CAD functionalities are not only images, i.e. image data, but also intermediate data that can be obtained during MRI scans, such as for example the total acquired resonance signal herein below defined as resonance signal and/or also NMR relaxation measurements or the like.

As regards objective criteria determining indications helping the diagnosis about the presence or absence of a rheumatic disease and/or the evolution degree of the disease, the present invention provides the apparatus to be provided with processing sub-sections extracting from images alternatively or in combination measures regarding the synovial inflammatory condition, dimensions and/or morphology of the cartilage and/or the presence and/or the state of damages to the bony tissue.

Particularly MRI scanning means are shaped and have such a dimension that allow the acquisition of MRI images from anatomical regions of any joints and particularly of the hand, wrist, ankle, elbow, foot, knee and shoulder.

It is possible to provide two kinds of apparati one of which is able to acquire images from anatomical regions of the knee, ankle, hand, foot, elbow, wrist and possibly the shoulder if the apparatus is of the open magnet type, i.e. with a C-shaped or overturned U-shaped section and the other one with a much more reduced dimension only for the hand, wrist, ankle and foot.

Thanks to this limitation, the apparatus is relatively small, inexpensive and easy to be installed, however allowing to treat the most common rheumatic diseases and having the greatest impact.

According to an advantageous characteristic the invention provides for the sub-section determining the synovial inflammatory condition to comprise means determining perfusion parameters of the paramagnetic contrast medium in the synovia and means for comparing said parameters with a reference value for discriminating the presence/absence of a synovial inflammatory condition and/or for the comparison with a reference scale for determining a parameter indicating the evolution degree of the synovial inflammatory condition, which reference value for discriminating the presence /absence of the synovial inflammatory condition and/or which reference scale for determining the evolution degree of the inflammatory condition are included in a database of known clinical cases.

According to an alternative embodiment that can be provided also in combination with the previous embodiment the sub-section determining the synovial inflammatory condition comprises means for determining numerical values of said synovial inflammatory condition both regarding the presence/absence of it and the evolution condition of the inflammation by means of the comparative analysis of the contrast of RM images such as the detection of maps in T1 or T2 or analysis of images by means of suitable contrast media, obtained at different stages of the inflammatory condition from images relevant to patients being part of the database of known clinical cases and/or from images relevant to previous indagations carried out on the same patient.

Obviously said techniques for identifying the synovial inflammatory condition can be used for determining numerical parameters describing the condition of any tissue present in the anatomical region under examination and that is reproduced in the acquired image.

An alternative third embodiment that can be used also in combination with one or more of the previous ones provides the sub-section determining the synovial inflammatory condition to comprise means for determining numerical values of said synovial inflammatory condition both regarding the presence/absence of it and the evolution condition of the inflammation by processing image data by means of classification and/or predictive algorithms, which have been trained (training and testing) by means of synovial image data of known clinical cases included in a database of known clinical cases.

Still an embodiment that can be provided individually or in combination with one or more of the previous embodiments provides a sub-section segmenting image data for recognizing and delimiting one or more image regions comprising subsets of pixels or voxels, measurements of parameters or of perfusion curves being carried out within one or more of said limited image regions.

As regards the sub-section determining dimensions and/or the morphology of the cartilage, particularly in the anatomical region of the hand and/or knee, the invention provides it to comprise means segmenting images for determining subsets of image data and/or pixels and/or voxels and for identifying real objects that are represented in the image by said subsets of image data and/or pixels and/or voxels, the subset of pixels or voxels or image data representing the cartilage being identified and dimensions and/or the shape of said cartilage being determined. Therefore dimensions and morphologic characteristics of the cartilage are determined on the basis of dimension and morphologic characteristics determined from images and particularly from the subset of pixels, voxels or image data that has been identified representing the cartilage in images.

The sub-system determining the dimensions and/or the morphology of the cartilage can further comprise means for generating a virtual image reconstructed on the basis of image data processed by segmentation means. These means are known as rendering means and the invention provides them to be possibly combined with morphing and/or smoothing means.

According to a further characteristic of the invention, the sub-section determining dimensions and/or the morphology of the cartilage in the anatomical region of the hand and/or knee comprises means for comparing dimension and/or morphologic data of the cartilage with dimension and morphologic data of a plurality of know clinical cases that are kept in a database of clinical cases. Said comparison provides information about the differences and indentities of dimensions and/or morphology of the cartilage with respect to dimensions and/or morphology of the cartilage of one or more known clinical cases, and therefore it provides an indication about the presence /absence of a rheumatic disease and/or an indication about the evolution condition of said rheumatic disease.

As regards the comparison reference numerical values are determined according to the invention from a database of known clinical cases. This database comprises images of the anatomical region of the hand and/or knee and dimension and/or morphologic data of the cartilage are determined from said images by means determining dimensions and/or the morphology of the cartilage in the anatomical region of the hand and/or knee working by processing image data by means of segmentation and/or rendering and/or morphing and/or smoothing. At least one or more reference values are determined for discriminating the presence/absence of a rheumatic disease and/or a scale of numerical values and/or a function correlating different dimension and/or morphologic conditions of the cartilage and different evolution degrees of the disease, whereas dimension and morphologic values of the cartilage for a case under examination which have been determined by the corresponding subset are compared with said reference value and with said scale or said correlation function for determining an indication about the presence/absence and/or the evolution degree of a rheumatic disease.

Still according to a further characteristic of the invention, the apparatus comprises a sub-system or a sub-section determining the presence and/or the condition of damages to the bony tissue, particularly in the anatomical region of the hand and/or of the knee, by means automatically or semi-automatically determining the amount of bony erosions and the volume thereof, as well as the ratio between the volume of bony erosions with respect to the volume of healthy bone.

The invention provides different means and different methods for determining above numerical parameters.

According to an embodiment means for automatically or semiautomatically determining the amount of bony erosions and the volume thereof, as well as the ratio between the volume of bony erosions with respect to the volume of healthy bone are composed of means segmenting images for determining subsets of image data and/or pixels and/or voxels and for identifying real objects that are shown in the image from said subsets of image data and/or pixels and/or voxels, the subset of pixels or voxels or image data representing the bony tissue being identified.

As in the previous case in combination with segmentation means it is possible to further provide means for generating a virtual image reconstructed on the basis of image data processed by segmentation means, such as rendering means possibly combined with morphing and/or smoothing means.

An alternative provides the sub-section determining the presence and/or the condition of bony tissue damages to comprise means determining dimensions and/or volume and/or the morphology of the bony tissue from subsets of pixels and/or voxels and/or image data that have been identified as representing the bony tissue in the body part under indagation, whereas erosions, dimensions and/or volume and/or the morphology thereof are determined by comparing dimension and/or volume and/or morphologic data of the bony tissue obtained by processing the MRI image or images of the body subjected to indagation with dimension and/or volume and/or morphologic data of a plurality of known clinical cases kept in a database of clinical cases, which comparison provides information about differences and identitites of dimension and/or volume and/or morphology of the healthy bony tissue and/or of erosions as regards dimensions and/or the volume and/or the morphology of the healthy bony tissue and/or of erosions of one or more known clinical cases, and therefore provides an indication about the presence /absence of a bony damage and/or an indication about the evolution condition of said damage i.e. a staging of the bony damage.

The ratio between the volume of erosions and/or the volume of healthy bony tissue is determined by determining the volume of erosions by the difference of dimensions and of the shape of the bony tissue as determined by images of the body under indagation with typical dimensions and the typical shape for example average dimensions and average conformation of the bony tissue, the volume of healthy bony tissue being determined by the difference of the erosion volume determined in such way and the average volume or typical volume for the bony tissue without a disease, data relevant to dimensions, volume and morphology of the bony tissue without a disease being determined by means of the database of known clinical cases.

According to an alternative the set of pixels or voxels or image data reproducing the image of the bony tissue in the MRI image is compared with the image of the same bony tissue obtained from dimension and/or volume and/or morphologic data of the database of known clinical cases, a morphing or registering process being carried out in order to bring said images of the bony tissue in congruence and which process provides numerical information about dimension, volume and morphologic differences between the bony tissue represented in the image of the body under indagation and the bony tissue having typical dimensions and/or volume and/or shape and relevant to the database of clinical cases, which numerical indications refer to deformations necessary to bring images of the bony tissue under congruence and registration and indicate a registration and/or congruence error constituting a measure of the bony damage.

Typical values of dimensions, volume and morphology of the bony tissue without a rheumatic disease with different evolution conditions of a rheumatic disease are set, the presence or absence of a disease and/or the evolution degree of the disease being determined by the comparison between typical values and corresponding values determined by the sub-section determining the bony damage or the presence/absence of the disease and/or the evolution degree thereof being determined by the morphing and/or registering process of images of the bony tissue corresponding to said typical dimension volume and morphologic values.

As regards means determining above numerical parameters, the image or images are often subjected to a segmentation and/or rendering process. The invention advantageously provides means verifying the quality of the segmentation and/or rendering process. This is a critical one and it has to provide reliable and precise data. Therefore according to a further characteristic of the invention, the apparatus comprises means for verifying the reliability in identifying the subset of pixels or voxels or image data representing in the image or in the set of image data a real object and particularly the cartilage and/or the bony tissue and/or restricted regions where the perfusion is evaluated. These verifying means comprise a database of dimension and/or volume and/or morphologic configuration data, i.e. of typical shapes of the cartilage and/or of the bony tissue and/or regions where the perfusion is evaluated under specific conditions of absence or presence of a rheumatic disease and/or a specific evolution degree of the rheumatic disease. Dimensions and/or volume and/or the morphology of the cartilage, of the bony tissue and/or of perfusion evaluating regions determined by sub-sections of means processing images of a patient under examination are compared with said typical dimension and/or volume data and/or with typical morphologies for the cartilage, for the bony tissue and for perfusion evaluating regions that are in the form of average values and/or a range of average values of dimension and/or volume and/or morphologic differences. In order to determine an objective result of the comparison a first maximum difference threshold is set, over which dimension and/or volume and/or morphologic data provided by sub-sections are considered as unreliable and incompatible ones. In such case dimension, volume or shape differences with respect to standard values are too much great and the probability of being errors or malfunctions is high. Verifying means are provided with a sub-section signalling or requiring the repetition of the process for determining dimensions and/or volume and/or morphologic characteritics and/or of the acquisition of MRI image or images or said verifying means are provided with a sub-section automatically commanding the repetition of the process for determining dimensions and/or volume and/or morphologic characteristics and/or the acquisition of MRI image or images.

It is possible to provide the process verifying the segmentation and/or the rendering to be iteratively repeated for a predetermined number of times that can be set as the user desires and/or till differences in dimension and/or volume and/or morphology came back within the maximum difference threshold.

The integration of means acquiring, generating and displaying MRI images with analysing means having CAD functionalities according to the present invention has a further considerable advantage that is the possibility of an interaction between means acquiring, generating and displaying MRI images and analysing means having CAD functionalities, according to which, analysing means with CAD functionalities comprise means for analysing acquired images with regards to predetermined quality parameters of said image data and/or of resonance signals and means for automatically changing acquisition settings and/or acquisition parameters of image data that are controlled by said means analysing image data on the basis of quality parameters of image data and/or of resonance signals from which they are determined.

It is well known that the excitation of resonance signals can occur according to different protocols that are characterized by different settings of parameters and different acquisition sequences. Parameter settings and the choice of one acquisition sequence or of the other one determines not only the quality of the image, as regards the resolution and/or contrasts and/or signal/noise ratio or the presence of artefacts, but also information that can be seen or extracted from acquired images and the length of the acquiring process.

According to the above characteristic of the interaction between purely tomographic means and means purely dedicated to the processing with CAD functionalities the optimization of all parameters aiming at the best final result can be also "guided" by CAD itself. That leads to the advantage of a better functionalities acquiring, generating and displaying images and a more reliable CAD functionalities, since means having CAD functionalities carry out the adjustment on the basis of specific requirements of said means with CAD functionalities for optimizing their task.

Therefore the invention provides the detecting section, particularly the unit detecting images by nuclear magnetic resonance to be connected to the section processing acquired images by means of a feed-back line.

The processing section controls the change of parameters setting the detecting section and/or the choice or the change of image acquisition sequences carried out by the detection section for acquiring images on the basis of a verification of the quality of image data and/or of resonance signals with reference to characteristics of image data and/or resonance signals important for carrying out the processing processes carried out by the processing section.

According to a further characteristic, the detection section by the feed-back line controls the processing section in treating image data and/or resonance signals by one or more different sub-sections and according to a predetermined order on the basis of the greatest quality that can be obtained from image data and/or signals and/or the length of the acquiring process, of the length of the processing process and of the reliability of results of the image processing expressed in the form of statistic reliability or error parameters and/or in the form of fitness.

In particular the apparatus comprises a unit generating setting parameters of the detection section, particularly of means acquiring MRI images and/or selecting and/or generating and/or shaping acqusition sequences, which unit can be controlled by a unit verifying the quality of image data and/or of resonance signals and/or evaluating statistic reliability or error parameters and/or in the form of fitness and which verification unit controls the unit generating setting parameters of the detection section, particularly of means acquiring MRI images and/or selecting and/or generating and/or shaping acquisition sequences to generate new setting parameters and/or choose and/or generate and/or shape new acquisition sequences on the basis of results of the quality verification of image data and/or resonance signals and/or evaluation statistic reliability or error parameters and/or in the form of fitness.

The verification unit can work in an iterative way and in this case it is provided with a counter for setting a maximum amount of activations for the control generating setting parameters of the detecting section, particularly of means acquiring MRI images and/or selecting and/or generating and/or shaping acquisition sequences and/or of means setting a minimum quality threshold of image data and/or of resonance signals and/or of error or statistic reliability and/or fitness of processing results.

The invention can provide also mathematical means such as for example fuzzy algorithms or genetic algorithms for determining new parameters or new settings acquiring signals and/or image data such as for example based on the processing by means of genetic algorithms or other similar algorithms, which algorithms can also be guided by statistic algorithms selecting or predicting new values of acquisition parameters or of acquisition sequences that have a greater probability in providing better image data from the point of view of processing means.

In all above cases, the automatic or semi-automatic definition of the processing result that is the auxiliary indication of the diagnosis occurs by the comparison with a reference database of data of a general record of cases and/or of the record of cases related to the specific patient.

For making said database, for example, data obtained by means for determining the inflammatory condition and/or the presence of damages to cartilages and/or to bony tissue can be associated to numerical variables whose values are defined with reference to a predetermined scale of numerical values and so can be used as variables for generating records that can be evaluated by expert systems or predictive systems, such as for example systems working on the basis of predictive algorithms of the artificial neural network type or statistic classifiers such as bayesian classifiers, Bayes networks, Support Vector Machines or the like.

From the above the synergic advantages are clear deriving from the fact the CAD system is integrated in the MRI system. By that the two systems can work in a mode optimized one with respect to the other.

Still according to a further advantageous characteristic, the system provides means storing a database of diagnostic images and of processing data with CAD means relevant to each patient.

Storing means can be composed of means integrated or resident in the system computer or also of movable and portable storage media in combination with readers of said media provided in the system.

In addition to diagnostic data i.e. to images and results of image processings, the patient database can comprise additional data, such as image acquisition settings, used processing means, conditions of the patient at each image acquisition defined on the basis of other physical or physiological parameters.

By means of the patient database, it is possible to repeat specific image acquisitions and corresponding processings with processing means having CAD functionalities in different time moments even at relatively long time intervals, such as days, months or years and to determine the evolution of a condition by the comparison of image data and/or of processing results. In the technical language this mode is known as follow up and it allows to verify the development of a therapy for example in order to determine the kind of intervention to be carried out and/or to verify the efficacy of a therapy in progress, for example by changing it in the case of a slow or inexistent development as regards recovery, such as for example changing the dosage of drugs and/or the kind of active principle. Similarly it is also possible to control the evolution of side effects of the therapy and to possibly act in limiting it or in reducing the dosage if side effects are worrying.

To this aim, the diagnostic imaging carried out on anatomical regions subjected to rheumatic diseases can be integrated with physiological data and/or images from other anatomical regions which can be acquired in a different form or by different acquisition methods and which anatomical regions are those in which or for which potential side effects are provided.

These data can also be stored in the patient database. That is possible by using data coding protocols known as DICOM and constituting a standard in the medical field.

When comparing MRI diagnostic images and results of their processing with CAD processing means first the system verifies acquisition settings and processing means used in the previous diagnostic image acquisition or acquisitions and processing or processings and it uses the same settings even for the new image acquisition and for processing them. Image data and/or processing results therefore are compared one with the other and a compatibility verification is carried out intended to indicate if the possible change of conditions determined by the subsequent examination, i.e. by the subsequent image processing and acquisition session, is consistent also with a drastic aggravation or with a drastic and sudden recovery and it indicates conditions of image data and results of their processing that can be consistent with or not consistent with image data and results of their processing relevant to a previous examination, advising or automatically carrying out a process repeating the acquisition of the image or images and the processing thereof in case with modified sequences and/or parameters and/or with processing means that are modified and/or different ones and/or integrated with one or more different processing means in addition to the already used ones.

Moreover a further characteristic of the invention is the fact of providing the integration of image data or other data obtained by the magnetic resonance technique with image data or other data obtained by different techniques acquiring diagnostic images, such as for example radiologic or ecographic techniques, or the like and that both as regards image data and/or other parameters directly deriving from acquired signals and/or as regards processing results of said image data and/or acquired signals.

Both for correctly carrying out the comparison in the follow up process and for the integration and/or merging of information obtained by different acquiring techniques it is important to provide means automatically or semiautomatically registering images that are generally also known.

In the present invention processing means are composed of one or more software modules that are independent and that can be combined in any combination and that can be interfaced and are composed of a software classificating and processing MRI image data, a 3D modelling and displaying software, a software for the quantitative analysis of specific parameters of the applicative method and a software for the decisional support in carrying out various steps of the examination and of the analysis of results. Preferably said softwares work on a standard platform (PC/Windows) and are loaded and are executed a processing hardware integrated in a dedicated MRI tomograph having characteristics optimized for such aim.

The invention relates also to a method for determining indications helping the diagnosis of rheumatic diseases comprising steps of acquiring one or more images by nuclear magnetic resonance and subjecting image data and/or resonance signals to a processing for determining from image data and/or resonance signals, values of one or more numerical parameters indicating of the presence or absence of a rheumatic disease and/or a measure of the evolution condition of said rheumatic disease, said processing being carried out immediately after the acquisition of the image or images under nuclear magnetic resonance.

Further characteristics of the method are described in subclaims of the method.

Further improvements of the invention are object of subclaims.

Characterstics of the present invention and advantages deriving therefrom will be more clear from the following description of some embodiments with reference to annexed drawings wherein:
Figure 1 is a block diagram of the principle structure of the system according to the present invention.
Figure 2 is an example for carrying out the system according to the present invention wherein means acquiring MRI images and means processing images with reference to CAD functionalities are composed of a software and hardware combination and particularly of applicative programs that are executed by a computer in common both to acquiring means and to processing means.
Figure 3 is a block diagram showing the principle of processing means for recognizing objects from the morphologic and/or dinamic-functional point of view.
Figure 4 is a block diagram showing an example of the segmentation process, wherein the traditional process segmenting the image is integrated with morphologic and/or dynamic functional data typical for real objects reproduced in images.
Figure 5 is a block diagram of means processing images that are specifically provided in the system according to the present invention for the diagnostic help by a computer in the rheumatic field.
Figure 6 is a block diagram of the system according to the present invention regarding an example of automatic or semiautomatic interaction means between means acquiring images and means processing images for optimizely setting particularly acquiring means with reference to processing means.

With reference to figures a diagnostic helping system in the rheumatic field by means of nuclear magnetic resonance image acquisition comprises an MRI apparatus for acquiring images, particularly diagnostic images, by nuclear magnetic resonance wherein a unit for processing acquired images is integrated providing to extract from images information about the state or conditions of objects reproduced in said images helping the diagnosis.

Particularly the system according to the present invention is intended for recognizing the presence/absence of rheumatic diseases and their evolution degree in the specific case of hand and knee regions. However above principles that will be described in more details can be applied to any anatomical regions subjected to rheumetic diseases.

At present different means for processing images are known per se having different functionalities and intended to define or to extract from image data specific characteristics of images and/or of objects reproduced in the image. These means are composed of algorithms processing images i.e. image data. In order to better understand the following description it is to be noted that digital or digitized images are composed of individual points called pixels in two-dimensional images and voxels in three-dimensional images. The visual information of the image is determined by the appearance of each individual pixel or voxel with respect to surrounding pixels or voxels. The appearance of each individual pixel or voxel can be defined by specific parameters that are univocally defined in colorimetric systems by using different kinds of definition spaces like the well-known ones defined for example as HSV or other ones.

Therefore the image is defined by a set of pixels or voxels ordered in the form of a two-dimensional array as regards pixels and a three-dimensional array as regards voxels. Therefore to each unit image element i.e. to each pixel or voxel there is associated a set of parameters describing their appearance. The set of these parameters that are grouped together in the form of a two-dimensional or three-dimensional array depending if they refer to pixels or voxels, describes and defines in the numerical form the two-dimensional or three-dimensional image and specific values of parameters correlated to each pixel or to each voxel are generated from signals riceived from a body under examination or a part of the body under examination by extraction functions that are defined by the kind of used signal and/or by the acquisition mode.

Therefore algorithms processing images work on said sets of image data in the form of two-dimensional or three-dimensional arrays and such operations are substantially equivalent or can be defined as operations carried out on the image since as mentioned above image data are only a numerical representation of the image.

Processing means with CAD functionalities can be advantageously composed of computer programs that execute operations defined by the algorithms processing images and which programs are executed by computers and particularly by Personal Computers or computers with similar system architectures.

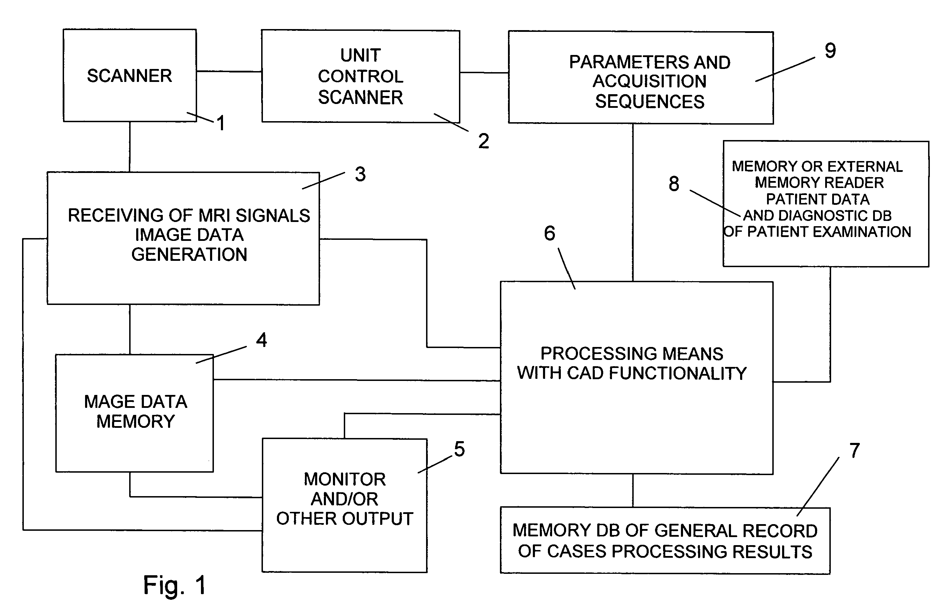

Figure 1 is a block diagram of a system according to the present invention. The system comprises a unit for acquiring images by nuclear magnetic resonance and a unit processing images with CAD functionalities both grouped together in a single apparatus.

The unit acquiring MRI images comprises a scanner 1 with a magnetic structure and with various gradient, transmitting and receiving coils, as well as with units generating image data from received resonance signals. Therefore the scanner 1 represents the magnetic structure, gradient coils, receiving coil and the transmitting one and further possible devices or means for acquiring resonance signals. 2 and 3 indicate means controlling the scanner and means receiving signals and generating image data and both are generally composed of electronic devices. These means 2 and 3 can be also composed of combinations of hardware and software means and as it will be seen below of software means loaded in a general hardware executing the software, such as a computer or also a personal computer.

Image data generated by the unit 3 are stored in a memory 4 and therefore they can be called up for being displayed at any moments by means of the monitor 5 or other output means or they can be immediately displaying apart from the storage.

The unit processing image data having CAD functionalities comprises processing means with CAD functionalities that are indicated by 6 and to which there are provided or which call up image data of one or more images to be subjected to processing processes from memory 4, or from the unit receiving and generating image data 3 and/or possibly even from the monitor or from further possible output means 5 when they allow it. As it will be seen below processing means can provide different kinds of outputs that as regards the form and contents depend on specific functional requirements. Processing results can be in the form of alphanumeric data and/or as an alternative or in combination in the form of static images and/or as an alternative or in combination in the form of dynamic images i.e. image sequences like films or the like. Results in the alphanumeric form and/or in the form of image or images can be displayed on the monitor 5 or printed or personnel can access to them by other output means. Moreover in combination or as an alternative results are stored in a memory 7 of general record of clinical cases that as it will be seen below has both the task of having data of various examinations for each patient available in order to allow the identification of the time evolution of patient conditions and the task of increasing a database of known record of cases that is necessary for training and testing expert algorithms used by processing means with CAD functionalities 6 and improving their performances in time and by the use by means of a constant learning. As an alternative or in combination it is possible to provide also a reading/writing unit 8 of an external portable memory, such as a tape, a floppy-disk, a writeble or rewritable CD or DVD or a so called smart card wherein data of each examination for each patient are stored together with other data obtained from other examinations.

Thanks to that it is possible to guarantee patient privacy, limiting data relevant to patients introduced in the database 7 of record of cases and keeping possible more sensitive and private information both personal and diagnostic ones only inside the portable memory in patient's hands, therefore the access to these data is legally possible only with the express will of the patient and only when the examination is carrying out. This mode can be carried out by means of the integration of means processing images with acquisition means and by the substantially immediate execution i.e. within the same examination session of said processing. Therefore, as it will be seen below, when means processing images provide the use of predictive or classification algorithms for image data for indicating and/or classificating predetermined searched characteristics of objects represented in images and particularly an indication of a probable presence or absence of a rheumatic disease and/or of the degree of such pathologic condition and/or the probability of developments with the disease improving or worsening, the use of data deriving from further different analyses or sources in combination with specific acquired images can be very important for the accuracy and reliability of the prediction and/or classification.

The practical realization out of the system according to the present invention requires the combination of hardware units with software units and such combination is particularly advantageous in the specific medical field relevant to diagnostic images.

As already said above, apparati for acquiring images in nuclear magnetic resonance are composed of combinations of operating units that are controlled by electronic control units, at least a part of said electronic control units being composed of one or more computer programs that are executed by a computer, that is made in particular with an architecture of the type similar to the one of personal computers or it is a real personal computer. In this case means for processing images having CAD functionalities can be composed of programs for processing images, i.e. image data that can be executed by a computer and particularly by a computer working in parallel to the one dedicated to the control of operating units for acquiring images or by the same computer.

Individual programs can be provided in the form of separated tools processing images or image data that can be used individually or as linked together according to different orders and in any combination or sub-combinations of different tools correspondingly to results desired from the processing. These tools can be considered as sub-sections or sub-systems of processing means that have an hardware-software combined structure.

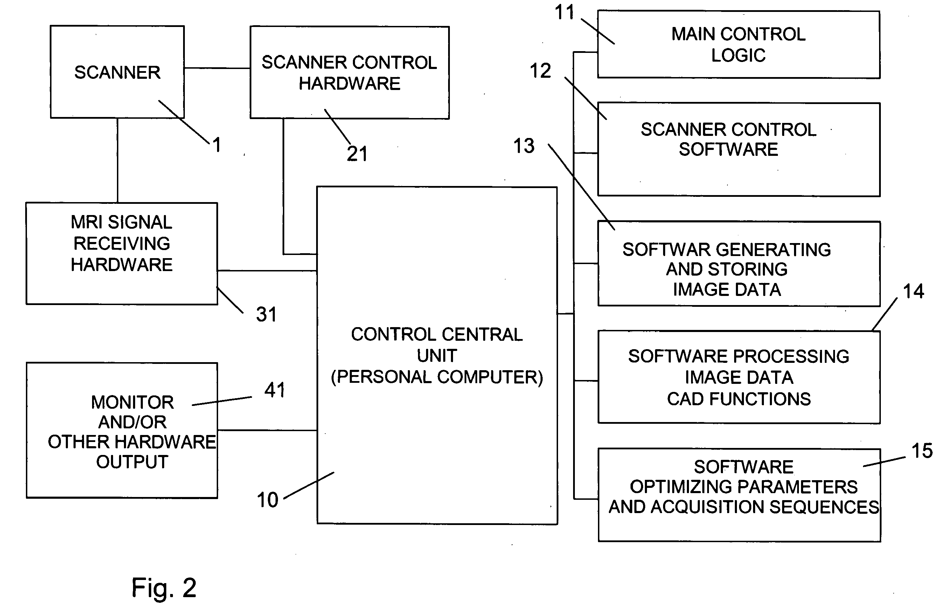

Figure 2 schematically shows an example of an apparatus incorporating the system of the present invention.

The apparatus provides a central control unit 10 composed for example of a personal computer or of two or more personal computers working in parallel one with the other.

In suitable memory areas or in dedicated memories there are stored different programs each of which is intended for executing specific tasks and is executed by the central unit 10 when such task is expressly required by a command of the personnel or when it is part of an operating sequence that is automatically executed and controlled by a control logic represented by the memory area 11.

The memory area 12 represents the program controlling the MRI scanner, such term scanner has to be intended in its wider meaning as already said above and so it comprises operating units mainly being the magnetic structure, gradient coils and the transmitting coil. Further operating units optional or having secondary importance are associated thereto. Moreover the scanner comprises also operating units necessary to detect and receive signals in this case magnetic resonance signals.

Operating units included in the scanner are controlled by hardware control units indicated by 21 and whose operation is controlled by a program controlling the scanner indicated by 12. Output resonance signals from the scanner are processed by a MRI signal receiving unit generating image data from MRI signals and comprising hardware means indicated by 31 and are controlled by a program generating and managing image data such as in particular the storage indicated by 13. Means processing images and/or image data are composed of one or more program modules that are executed by the same central control unit 10.

Image data that are not subjected to processing and/or processing results having CAD functionalities both in the form of alphanumeric indications and in the form of modified or reconstructed images are emitted by means of output devices 41, such as for example one or more monitors and are controlled by the central unit 10 and by special programs controlling the output devices.

With reference particularly to figures 1, 2 and 6 there is provided an interaction made by means of a feedback line between means acquiring MRI images, i.e. the portion of the apparatus for detecting MRI images and means processing images and/or corresponding image data with CAD functionalities in order to determine indications helping the diagnosis from images and/or corresponding image data. It allows to obtain an adaptation and/or a direct optimization between means acquiring images and means processing them, such to set at best means acquiring images with reference to optimal requirements of processing means.

Similarly to what described above means for the interaction between means acquiring images and means processing them are also composed of a combination of hardware and software means.

Referring to figure 1 showing a principle diagram of a system according to the present invention, there is provided a unit generating and/or setting parameters and nuclear magnetic resonance image acquisition sequences that is indicated by 9 and receiving setting command or setting changing command signals as input on whose basis it generates parameters setting the scanner 1 and/or it defines image acquisition sequences and it provides said parameters and said sequences to the unit 2 controlling the scanner for acquiring images.

With reference to figure 2 and as already mentioned above said unit 9 is composed of a combination of hardware means and software means. Considering the example of hardware/software architecture of figure 2 for the system according to the present invention in a memory or in a dedicated memory area there is stored a software optimizing parameters and acquisition sequences indicated by the box 15. Such software can be executed by the central control unit 10 managing both the interface between the software processing image data having CAD functionalities 14 with said software optimizing acquisition parameters and acquisition sequences 15 and the generation and sending of setting or changing setting commands of parameters and acquisition sequences to the unit 21 controlling the scanner 1.

It is possible to provide different modes for specifically carrying out the interaction between the image acquiring apparatus and image processing apparatus with CAD functionalities above all as regards criteria on the basis of which acquisition parameters are determined or changed.

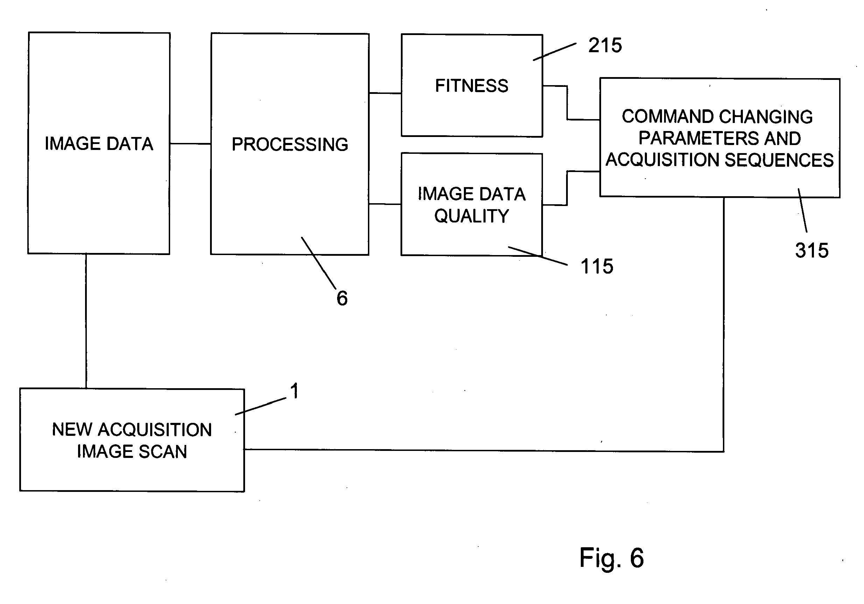

Figure 6 is a block diagram of a sub-system for the interaction between the apparatus acquiring images and means processing them.

Image data from the receiving unit 3 are provided to the processing unit 6. As it will be more clear below, CAD processing means obtain information from image data by using algorithms of the statistic, predictive, or evolutive type that however provide also reliability or error parameters of output data together with output data. Moreover resonance signals from which image data are taken can be analyzed as regards their quality with reference to some quantities important for the image quality and particularly they are the signal/noise ratio, resolution, contrast but are not limited thereto.

Therefore interaction means can have two or more different sections determining parameters and acquisition sequences one of which working on the basis of reliability parameters of the output of CAD processing means and the other one working on the basis of quality parameters of resonance quality and/or image data such as indicated by 115 and 215. Therefore the two sections provide information regarding the quality of image data with reference both to mere acquisition steps and to the reliability of output data of processing means, therefore in the quality verification section 315 by the comparison with threshold values for relative reliability parameters and/or for quality parameters of resonance signals and/or of image data indications are provided about the fact if it is necessary to provide the definition of new values of setting parameters and/or the change of image acquisition sequences and therefore to send a command to the unit generating acquisition parameters and/or sequences represented by the optimization software. On the basis of the command of the verification section the optimization software i.e. the generating section provides to generate modified setting parameters and/or to change only the used sequences.

Therefore such new setting parameter values and/or such changes of image acquisition sequences are provided to the scanner 1 for carrying out a new image acquisition scan.

Image data obtained by the new scan can be subjected again to steps described above for verifying if changes of settings have lead to expected improvement results with regards to the quality of resonance signals and/or image data and in combination or as an alternative with regards to reliability of outputs of processing means.

Above steps can be also iteratively repeated till a certain amount of iteraction steps is carried out and/or till the quality of resonance signals and/or image data and/or the reliability of outputs of processing means reach or overcome predetermined minimum threshold values.

As regards changing values of setting parameters and/or the choice or structure of acquisition sequences, such values or such data can be determined also by using statistic evolution systems, such as genetic algorithms or other similar algorithms by means of which new setting parameters and/or new acquisition sequences are determined that are then used for steps acquiring resonance signals and image data.

With reference to setting parameters and/or to acquisition sequences determined on the basis of feedback by processing means as indicated above, and especially if such values or such sequences are determined by evolutive algorithms, so it is possible to introduce a test acquisition step wherein a reduced amount of resonance signals is acquired or image data or sequences are carried out for a limited field of vision with respect to the desired one, in order to reduce acquisition time. Resonance signals and/or image data obtained by said test acquisition steps are therefore subjected to quality control and the obtained quality is compared with the one obtained in the scan of the preceding acquisition step. When setting parameters or sequences used in the test acquisition step provide quality improvement with respect to the ones of preceding acquisitions therefore the complete acquisition step is carried out, whereas when such quality improvement of resonance signals and of image data is not detected the step computing new acquisition parameters or new sequences is again carried out without carrying out the complete image acquisition step and so drastically reducing test time.

The above allows to obtain a direct and immediate interaction between acquisition means and processing means with processes iteratively carrying out the optimization of acquisition means with respect to processing means and to results provided therefrom. It is also possible for the interaction to occur in a bidirectional way. Particularly when processing means require such a setting of acquisition parameters and/or of sequences to determine acquisition times that are very long or greater than specific thresholds, therefore the acquisition apparatus communicates to CAD processing means that required acquisition parameters and/or sequences cannot be executed and it requires a change of processing means as regards carrying out particular processes treating image data and/or resonance signals that usually are not carried out, such as not linear filters or other image treatment processes, by means of which the extension of overall examination time is greater than time provided on average, but smaller than the ones that would be necessary by changing acquisition parameters and/or sequences such as required by processing means in the configuration conventionally provided.

Such condition can be easily carried out, since as it will be more clear below and however such as it already results from figure 2, processing means are composed of a plurality of software tools or modules that can be independently addressed one with respect to the other and that can be interfaced one with the other in any combinations and amount as regards operations to which image data and/or resonance signals are desired to be subjected.

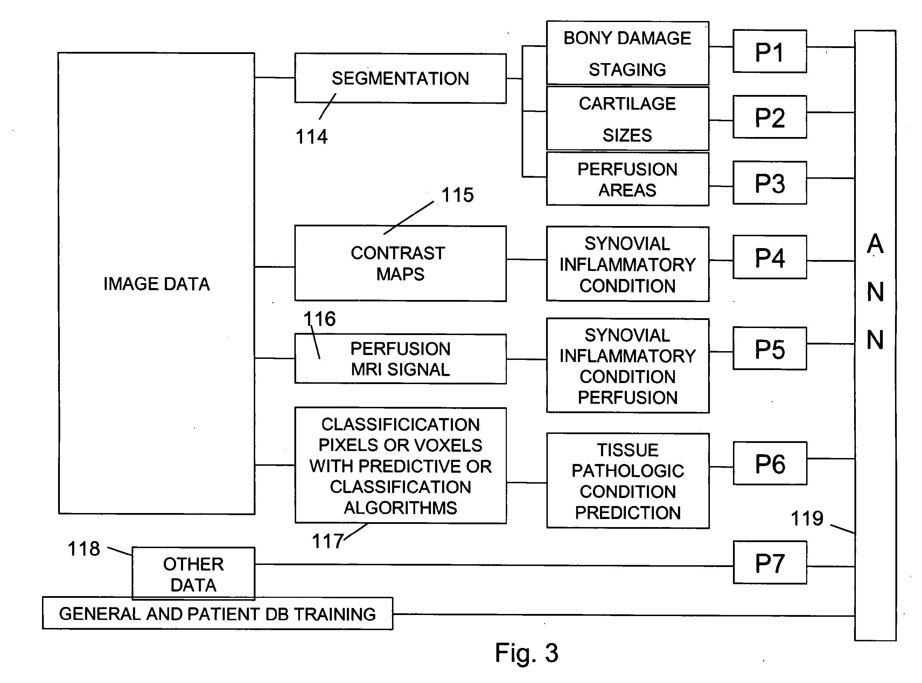

Figures 3 to 5 schematically show an example of different processes treating resonance signals and/or image data that can be provided in combination one with the other such as tools (instruments) of processing means.

In such figures reference will be made to particular methods treating images working on the basis of different algorithms types. These algorithms and the corresponding application to the general image treatment is known per se and below there is provided a list of pubblications wherein different types of algorithms and methods for processing images are described in details, i.e. different image processing tools whose specific structure is not the object of the present invention that supposes its existence.

A more detailled description of digital image processing on image segmentation can be found in:
1. Middleton I, Damper RI. Segmentation of magnetic resonance images using a combination of neural networks and active contour models, Med. Eng Phys 2004; 26:71-86.
2. Grau V, Mewes AU, Alcaniz M, Kikinis R, Warfield SK. Improved watershed transform for medical image segmentation using prior information. IEEE Trans Med Imaging 2004; 23:447-458.
3. Lucier BJ, Kallergi M, Qian W, DeVore RA, Clark RA, Saff EB, Clarke LP. Wavelet compression and segmentation of digital mammograms. J Digit Imaging 1994; 7: 27-38.

The image registration is described in more details in:
1. Sorzano, CO, Thevenaz P, Unser M. Elastic registration of biological images using vector-spline regularization. IEEE Trans Med Imaging 2005; 52:652-663.
2. Crum WR, Hartkens T, Hill DL. Non-rigid image registration: theory and practice. Br. J Radiol 2004; 77 Spec. No 2:S140-53
3. Park H, Bland PH, Brock KK, Meyer CR. Adaptive registration using local information measures. Med Image Anal 2004; 8:465-473.
4. Kim J, Fessler JA. Intensity-based image registration using robust correlation coefficients. IEEE Trans Med Imaging 2004;23:1430-1444
5. Pluim JP, Fitzpatrick JM. Image registration. IEEE Trans Med Imaging 2003; 22:1341-1343.

Curves picking up contrast media and methods for determining said curves are described in:
1. Daldrup-Link HE, Brasch RC. Macromolecular contrast agents for MR mammography: current status. Eur Radiol 2003; 13:354-365.
2. Sardanelli F, Iozzelli A, Fausto A. Contrast agents and temporal resolution in breast MR imaging. J Exp Clin Cancer Res 2002; 21:69-75
3. Baum F, Fischer U, Vosshenrich R, Grabbe E. Classification of hypervascularized lesions in CE MR imaging of the breast. Eur Radiol 2002; 12:1087-1092
4. Turetschek K, Roberts TP, Floyd E, Preda A, Novikov V, Shames DM, Carter WO, Brasch RC. Tumor microvascular characterization using ultrasmall superparamagnetic iron oxide particles (USPIO) in an experimental breast cancer model. J Magn Reson Imaging 2001;13:882-8
5. Ercolani P, Valeri G, Amici F Dynamic MRI of the breast. Eur J Radiol 1998;27 Suppl 2:S265-71
6. Tofts PS. Modeling tracer kinetics in dynamic Gd-DTPA MR imaging. J Magn Reson Imaging JID - 9105850 RN - 0 (Contrast Media) RN - 0 (Organometallic Compounds) RN - 0 (Radioactive Tracers) RN - 67-43-6 (Pentetic Acid) RN - 80529-93-7 (Gadolinium DTPA) 1997;7:91-101.
7. Griebel J, Mayr NA, de Vries A, Knopp MV, Gneiting T, Kremser C, Essig M, Hawighorst H, P.H. L, Yuh W. Assessment of tumor microcirculation: a new role of dynamic contrast MR imaging. J. Magn Reson Imaging JID - 9105850 RN - 0 (Antineoplastic Agents) RN - 0 (Contrast Media) RN - 0 (Radioactive Tracers) RN - 7440-54-2 (Gadolinium) 1997;7:111-9.
8. Hoffman U, Brix G, Knopp MV, Hess T, lorenz WJ. Pharmacokinetic mapping of the breast: a new method for dynamic MR mammography. Magn Reson Med 1995;33:506-14.

The use of classification algorithms and particularly the use of artificial neural networks, as well as systems coding pixels or voxels for procesing an image by means of a classification algorithm are described in:
1. Szabo BK, Wiberg MK, Bone B, Aspelin P. Application of atificial neural networks to the analysis of dynamic MR imaging features of the breast. Eur Radiol 2004;14:1217-1225.
2. Szabo BK, Aspelin P, Wiberg MK. Neural network approach to the segmentation and classification of dynamic magnetic resonance images of the breast: comparison with empiric and quantitative kinetic parameters. Acad Radiol 2004;11:1344-1354.
3. Vomweg TW, Buscema M, Kauczor HU, Teifke A, Intraligi M, Terzi S, Heussel CP, Acehnbach T, Rieker O, Mayer D, Thelen M. Improved artificial neural networks in prediction of malignancy of lesions in contrast-enhanced MR-mammography. Med Phys 2003;30:2350-2359
4. Perez de Alr, Ruiz-Cabello J, Cortijo M, Rodriguez I, Echave I, Regadera J, Arrazola J, Aviles P, Barreiro P, Gargallo D, Grana M. Computer-assisted enchanced volumetric segmentation magnetic resonance imaging data using a mixture of artificial neural networks. Magn Reson Imaging 2003;21:901-912.
5. Lucht RE, Knopp MV, Brix G Classification of signal-time curves from dynamic MR mammography by neural networks. Magn. Reson Imaging 2001;19:51-7
6. Markopoulos C, Kouskos E, Koufopoulos K, Kyriakou V, Gogas J. Use of artificial neural networks (computer analysis) in the diagnosis of microcalcifications on mammography. Eur J Radiol 2001;39:60-5
7. Vergnaghi D, Monti A, Setti E, Musumeci R. A use of a neural network to evaluate contrast enhancement curves in breast magnetic resonance images. J Digit Imaging 2001;14:58-59
8. Adbolmaleki P, Buadu LD, Maderimansch H, Feature extraction and classification of breast cancer on dynamic magnetic resonance imaging using artificial neural network. Cancer Lett 2001;171:183-91
9. Chen DR, Chang RF, Huang YL, Chou YH, Tiu CM, Tsai PP. Texture analysis of breast tumors on sonograms. Semin Ultrasound CT MR 2000; 21:308-316
and more generally in:
1. Buscema M. A brief overview and introduction to artificial neural networks. Subst Use Misuse 2002; 37:1093-1148
2. Haykin S. Neural Networks:A Comprehensive Foundation, 2 ed. New York: Macmillan 1999
3. Buscema M. Artificial Neural networks and complex social systems. I. Theory. Subst Use Misuse JID - 9602153 1998;33:v-xvii, 1-220 FAU - Bu
4. Buscema M. Theory: Foundation of Artificial Neural Networks. Substance Use & Misuse 1998;33:28-98.

As regards other processes for processing digital images known as morphing and rendering ones it is possible to generally refer to documents:
1. Digital Image Processing Jdhne Bernd Springer 2005.
2. 3-D Image Processing Algorithms, Nikoladis, Nikos; Pitas Ioannis, Wiley-Interscience 2000.
3. Non linear Model-Based Image/Video processing and Analysis, Pitas, Ioannis; 2001 Wiley-Interscience.

As regards specific CAD analysis means for images constisting in Bayesian classifiers and so called Support vector machines, in this case they are linear classification machines known for example from Nello Cristianini and John Shawe-Taylor. An introduction to Support Vector Machines and other kernel-based learning methods, Cambridge University press 2000 ISBN 0-521-78019-5 Chih-Wei Hsu, Chih-Chung Chang and Chih-Jen Lin, Practical Guide to Support Vector Classification Department of Computer Science and Information Engineering, National Taiwan Univeristy 2001 Taipei 106, Taiwan that can be downloaded from www.csie.ntu-edu.tw/cjlin/libsvm.

Therefore, with reference to figure 3, the system according to the present invention provides means processing image data having CAD functionalities composed of different software modules or programs each of which is a processing tool allowing to measure or determine parameters indicating the rheumatic disease and the evolution stage thereof.

By means of a segmentation module image data and/or corresponding pixels or voxels are divided in subsets, each of which is relevant to a specific object having its own identity independent of sets of image data and/or of pixels or voxels and each of which subsets of image data, pixels or voxels is the reproduction in the image of an independent or individual object being part of the body part or area under examination.

The segmentation allows to mainly define three parameters and i.e. the staging of the bony damage due to the rheumatic disease, the dimensions of the cartilage and to delimit circumscribed regions about which perfusion measurements have to be carried out.

Particularly for perfusion measurements described in details in EP 1.516.582, it is possible to use the segmentation of image or images since it allows to find up-take regions and the measure of the signal trend in these regions at subsequent times, in order to create curves of the signal of up-take region with respect to time starting from the injection of the contrast medium. Such curves are made as much precise as possible by the three-dimensional segmentation of all up-take regions and by the measure of the RM signal of such regions. Measures can be analyzed by automatic algorithms having the aim of defining characteristic parameters of such perfusion, such as for example the up-take rate, the possible stationary condition and the wash-out rate, that as it will be described in more details below will be correlated to other clinical parameters in order to define correlations with the pathologic condition and its potential evolution.

Segmentation is an image processing method known per se and it will be briefly summed up in the following description in order to make the comprehension easier with regards to the integration of this processing tool in the system of the present invention and for obtaining diagnostic quantitative information.

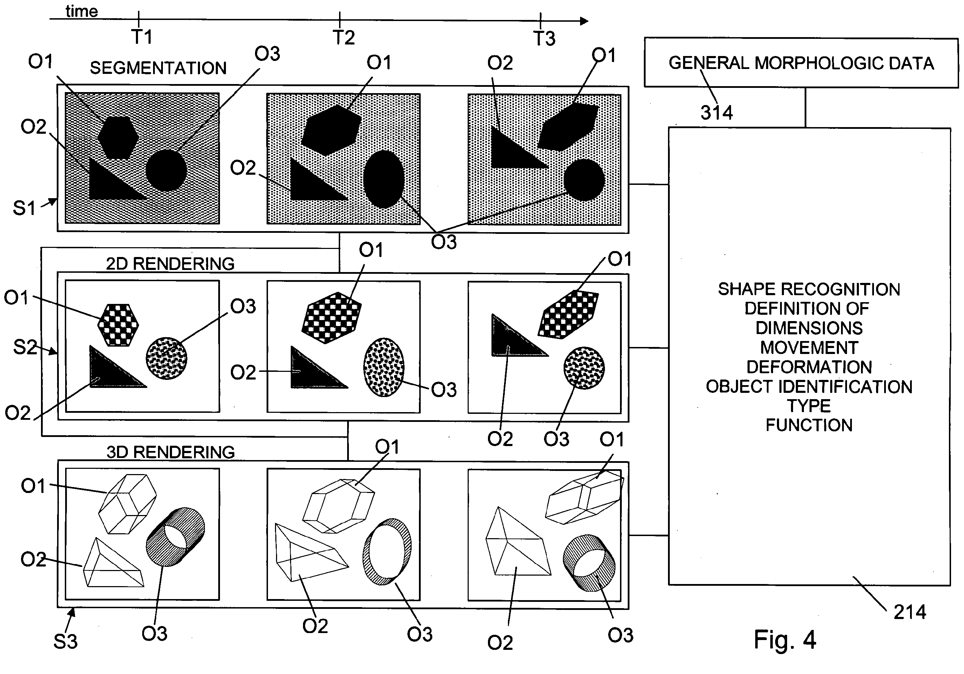

With reference to figure 4, it schematically shows the process for segmenting image data and allowing to carry out subsequent steps recognizing shapes of reproduced objects, dimensions thereof and/or movements and possibly also deformations.

S1 denotes a sequence of images acquired in different subsequent time moments indicated by T1, T2, T3. In this case they are images of the same anatomical region that have been acquired along the same scanning plane for 2D images or within the same scanning volume for 3D images.

As it is schematically indicated there are provided three objects 01, 02, 03 in the region that move and/or are subjected to deformation with the time goes by and representing in images three different objects provided in the volume of the body under examination subjected to image acquisition. These real objects for example can be three different kinds of tissue and/or three different kinds of organs or the like.

Segmentation is the process for defining subsets of pixels or voxels of images that are in regions or volumes corresponding to the reproduction in the image of the real object. Once said subsets have been defined it is possible to transform each subset in a virtual object therefore having its functional or semantic unit and that is the image of a real object comprised in the plane or volume of which the image has been acquired.

Therefore in each image it is possible to recognize the object and by the comparison with one or more preceding images it is possible to determine the behaviour in time of each object as regards the position, orientation, shape and dimensions.

Once objects and the behaviour in time thereof have been defined as said above, it is possible to provide the generation of a virtual image a kind of virtual copy of the real world wherein objects and behaviours are further highlighted by means of rendering, morphing, smoothing processes and other methods generating virtual realities.

Sequences S2 and S3 schematically show an example of 2D and 3D rendering of the behaviour of objects recognized in the sequence S1 by means of segmentation.

It is to be noted that while in segmented images, contrasts are still defined in the form of real image along the scanning plane or in the volume subjected to examination, i.e. contrasts and the appearance of pixels is directly determined by acquired signals, in images generated by rendering, morphing, smoothing processes or the like, it is possible to change the pixel appearance according to any univocal criteria and particularly in order to make easier the visual reading of the dynamic behaviour of objects and/or their topologic and dynamic interralations for the human user and that is by defining colorations and intensity changes according to different functions correlating image data and/or by means of smoothing contours and/or by introducing three-dimensional displaying effects such as for example shadings or light blazes and shades of colours.

Rendering and segmentation aim also at making the reading easier, both the automatical and the manual one, i.e. by the user for example of dimensions of the cartilage and/or stratifications of bony damages and/or dimensions, shape of circumscribed perfusion measuring areas.

By means of segmentation and rendering steps in combination possibly with morphing and/or smoothing treatments, it is possible to univocally give each subset of pixels or voxels to a real object, therefore generating from subsets of pixels or voxels representing the object in said image corresponding virtual objects that are considered as a single set in the following processing and allowing to determine numerical parameters regarding position, orientation, shape, movement in time and shape changes in time of various objects. Moreover by considering that generally reproduced objects are provided with a standard shape from which they are different for secondary variants, it is also possible to provide the comparison of morphologic and dimension data of various objects as determined by images by means of above processes with morphologic and/or dimension data typical of real objects therefore allowing to determine also the kind of object reproduced in the image by each subset of pixels or voxels.

Each object, such as for example each anatomical part has its own individual morphologic and dimension characteristics that by comparing them with shapes and dimensions of objects determined by images allow to verify if the object reproduced in the image coincides with the comparison one therefore also the identity or the kind of the obejct can be determined and possibly morphologic and dimension differences can be determined.

In figure 4 processes for recognizing shapes, for determining dimensions, the movement, orientation and shape changes, as well as the identification of real objects reproduced by virtual objects with reference to the kind and the task of these real objects are indicated by a subset 214, while the latter is interfaced with a further subset 314 providing morphologic and dimension data typical of real objects that can be compared with the ones determined in images.

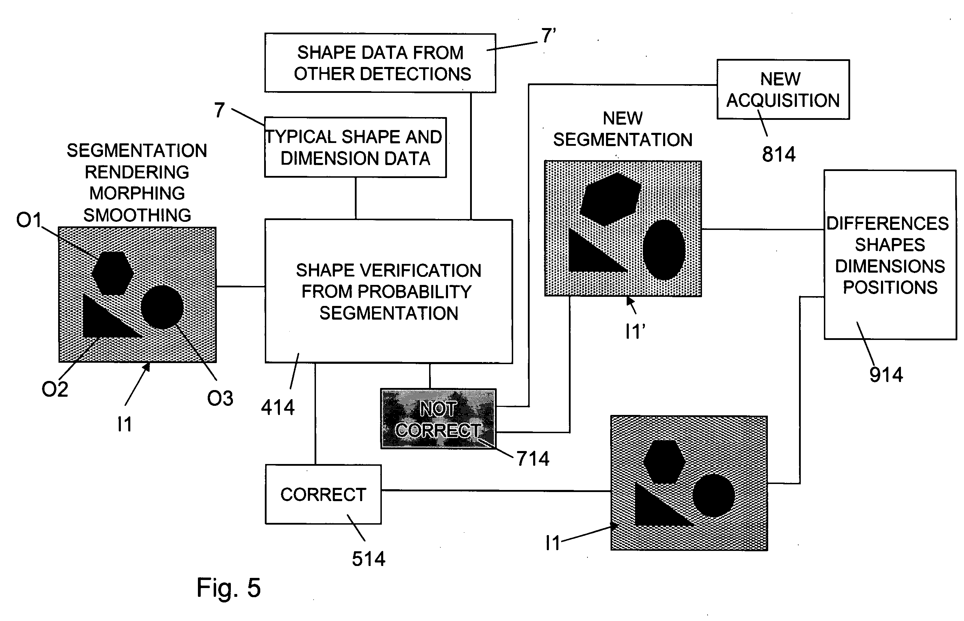

Figure 5 shows a further system for verifying the segmentation and the generation of renderized images as regards the compatibility of the morphology and dimensions of virtual objects identified in images with morphology and/or dimensions typical of corresponding real ones possibly also with reference to morphologic and dimension changes caused by condition changes as in the present case by the presence of a rheumatic disease.

In this case the image segmented and possibly further subjected to reconstruction by rendering possibly in combination with morphing or smoothing treatments, is analyzed with reference to the shape and dimensions of objects 01, 02, 03 identified in said image I1 and possibly also of topologic and dimension relations of said objects one with respect to the other in a verification unit 414. To this unit 414 there are provided morphologic and/or dimension data typical of objects considered as to correspond to the ones reproduced in the image I1 and indicated by 01, 02, 03. Such data can be relevant both to an average value and/or to a range included between minimum and maximum values. Moreover typical data can also consider values that are not standard and corresponding to typical pathologic conditions. Processing means can comprise such data inside the database of clinical cases stored for example in the memory or memory area 7 as indicated in figure 1.

Moreover it is possible to make the comparison with morphologic, topologic and dimension data obtained by other measuring or analysing methods, such as by means of other image acquiring means different from the magnetic resonance such as ultrasound, radiologic means etc. said data being also included in the database of clinical cases and being stored in a dedicated memory area indicated by 7' in figure 5.

When the result of the verification system denotes that morphologic and/or dimension and/or topologic data of objects obtained from corresponding virtual objects are compatible with corresponding typical data it is possible to go on as indicated by the box 514 and by the image I1 and in this case for example it is possible to determine shape dimension and position differences of real objects determined by the corresponding virtual objects with respect to corresponding shape, dimension and position data of the same real objects as provided by the database of clinical cases 7 and 7' as indicated by the function box 914. Moreoever these differences can be used as a standard criterion for defining the existence of a patologic condition considering the rheumatologic point of view and/or for evaluating the evolution degree of the disease if it is present. It is possible to define different numerical comparison parameters according to different comparison functions and constituting numerical values. The comparison of these numerical values of comparison parameters with predetermined threshold values empirically defined on the basis of known clinical cases already allow to define a diagnostic indication.

When the verification unit 414 establishes that there is no compatibility between morphology and/or dimensions and/or position of real objects determined by virtual objects with respect to shape, dimensions and positions of real objects obtained by the database of clinical cases, so image data I1, segmented and/or further renderized and/or possibly subjected also to morphing and/or smoothing are considered as wrong ones 614 and it is possible both to repeat the segmentation and/or rendering process and/or possibly the morphing and/or smoothing process as indicated by the image I1' or even to provide a new acquisition of the image from the body under examination as indicated by 914.

However it is to be noted how all function boxes 4141, 814, 914 are composed of program modules that are executed or can be executed upon call-up from the central processing unit and resident in a memory thereof or can be loaded in said memory.

As regards measures provided to be taken from image data and considered as measures indicating the presence or absence of a rheumatic disease and/or the evolution degree thereof the system according to the invention provides to carry on measures evaluating the inflammatory condition of the synovia and to identify and quantify possible damages to the cartilage or to bony tissue.

Quantitative information highlighting the inflammatory condition, can be obtained in a case by measuring typical parameters of the perfusion of the paramagnetic contrast medium in the synovia. Such method is known and it is described in more details in a previous application to the same applicant.

As an alternative or in combination it is also possible to determine a quantitative measure correlated to the inflammatory condition by the comparative analysis of the contrast of RM images. A particular example is the detection of maps in T1 or T2 obtained at different stages of the disease.

Damages to the cartilage can be quantized by measuring the cartilage, as regards thicknesses or overall volume. From these measures the pathologic condition can be detected, and by repeating at time intervals the examination as well as by comparing results of different examinations, the evolution during the therapy can be detected.

Segmentation tools and rendering tools in combination in turn combined with further morphing and/or smoothing processes allow to create tools segmenting the bony tissue from which a staging of the bony damage can be obtained by the fact that erosions are automatically or semiautomatically highlighted with reference to the number and volume thereof with respect to the volume of the healthy bone.

Finally it is also possible to process images simply by means of an analysis as regards RM contrast by means of which it is possible to automatically highlight pathological regions.

Figure 3 shows all such steps for processing image data. The function block of contrast maps is indicated by 115 with which the synovial inflammatory condition is evaluated as an alternative to the conventional perfusion measure indicated by the function block 116. The contrast map block 115 comprises means for determining the condition of tissues in the anatomical region subjected to examination and that are reproduced in the image which means as an alternative or in combination are composed of all means that are currently known in the examination field by nuclear magnetic resonance and that for example are composed of an analysis based on the detection of maps T1 and/or T2 or an image analysis by contrast media, or also by an analysis of the MRI signal as the ones obtained within the relaxometry examinations in nuclear magnetic resonance. Function block 117 represents the analysis as regards contrast, that specifically is indicated as a process for processing images of the pixel by pixel or voxel by voxel type and wherein algorithms can be linear or not linear filters such as edge detection filters, or classification algorithms that are linear or not linear such as bayesian networks or artificial neural networks or other algorithms of the evolutive type or combinations of such algorithms.

With a particular reference to means and to methods for determining numerical objective parameters measuring the presence or absence of the rheumatic disease and/or of the evolution stage thereof, the invention provides different possibilities that can be carried out individually or in combination one with the other.

As already mentioned a criterion provided by the invention is to determine a measure for the presence/absence and/or for the evolution degree of a synovial inflammation.

According to a first variant the synovial inflammatory condition is determined by measuring perfusion parameters of the paramagnetic contrast medium of the synovia. Such parameters or perfusion curves are compared with a reference value for discriminating the presence/absence of a synovial inflammatory condition. It is possible to provide a scale of perfusion numerical values describing different stages of the inflammatory condition therefore the comparison can also provide indications about the evolution degree of the inflammation which are in the form of a numerical parameter indicating the evolution condition of the synovial inflammation condition. The reference value for discriminating the presence/absence of the synovial inflammatory condition and/or the reference scale determining the evolution condition of the inflammatory condition are included in a database of known clinical cases and are determined in a way similar to the analyzed case by the same technique measuring the perfusion.

The synovial inflammatory condition can be also determined by means of the comparative analysis of the contrast of RM images such as the detection of maps in T1 and T2, obtained at different stages of the inflammatory condition from images of patients being part of the database of known clinical cases and/or images relevant to preceding indagation made on the same patient.

As an alternative the synovial inflammatory condition can be determined by processing image data by classification algorithms and/or predictive algorithms, which have been trained (training and testing) by image data relevant to the synovia of known clinical cases included in a database of known clinical cases.

A further objective criterion providing numerical description data that can be used for determining the presence/absence of a rheumatic disease and/or for determining the evolution degree thereof provides to determine dimensions and/or morphology of the cartilage in the anatomical region of the hand and/or the knee.

As already seen above in order to determine measures of the dimension and/or morphologic characteristics of the cartilage from image data it is necessary to previously subject images to an image segmentation process. It allows to determine subsets of image data and/or pixels and/or voxels and for identifying real objects represented in the image by said subsets of image data and/or pixels and/or voxels. Then it is provided to identify subset or subsets of pixels or voxels or image data representing the cartilage and finally to determine numerical values measuring dimensions of the cartilage and/or its shape as a function of dimensions and/or shape of said cartilage in the representation of the subset or subsets of pixels. Before measuring and/or identifying subsets of pixels or voxels or image data with image portions representing the cartilage it is possible also to subject segmented image data to a further processing process called rendering and substantially it provides the generation of a virtual image reconstructed on the basis of image data processed by segmentation means. The rendering process can be possibly combined to further image treating steps such as the so called morphing and/or smoothing one.

The presence or absence of the rheumatic disease and/or the evolution degree of said disease is obtained by comparing dimension and/or morphologic data of the cartilage determined by image data of the patient under examination with dimension and/or morphologic data relevant to a plurality of known clinical cases included in a database of clinical cases. The comparison can be made by different functions and it gives information about differences and identities of dimensions and/or morphology of the cartilage in the case under examination as regards dimensions and/or morphology of the cartilage of one or more known clinical cases, and so a numerical quantitative indication about the presence/absence of a rheumatic disease and/or an indication about the evolution condition of said rheumatic disease.

Still as regards the definition of dimension and/or morphologic characteristics of the cartilage, from a database of known clinical cases comprising images of the anatomical region of the hand and/or knee by means of means determining the dimensions and/or morphology of the cartilage in the anatomical region of the hand and/or knee working by a segmentation and/or rendering and/or morphing and/or smoothing processing of image data there are defined dimension and/or morphologic data of the cartilage, at least a reference value being generated for discriminating the presence/absence of a rheumatic disease and/or a scale and/or a function correlating different dimension and/or morphlogic conditions of the cartilage and different evolution degree of the disease being generated, whereas dimension and morphologic values of the cartilage for a case under examination and determined by the corresponding subset are compared with said reference value and with said scale or said correlation function for determining an indication about the presence/absence and/or the evolution degree of a rheumatic disease.

With reference to the definition of objective parameters for the presence/absence and/or evolution degree of the rheumatic disease, by detection and measurement of erosions of the bony tissue, the invention provides means for automatically or semiautomatically determining the amount of bony erosions and the volume thereof, as well as the ratio between the volume of bony erosions with respect to the volume of healthy bone. These means comprise means for segmenting images for determining subsets of image data and/or pixels and/or voxels and for identifying real objects that are represented in the image by said subsets of image data and/or pixels and/or voxels, the subset of pixels or voxels or image data representing the bony tissue being identified. Possibly in addition to the simple segmentation image data can be subjected also to the rendering and/or morphing and/or smoothing process as already mentioned above.

Dimensions and/or shape and/or volume of the bony tissue is determined by dimensions, volume and shape of subsets of pixels and/or voxels and/or image data identified as representing the bony tissue in the body part subjected to examination. Erosions, i.e. dimensions and volume and the morphology thereof are determined by comparing dimension and/or morphologic data of the bony tissue obtained by processing the MRI image or images of the body subjected to examination with dimension and/or morphologic data of a plurality of known clinical cases included in a database of clinical cases. By such comparison information about differences and identities of dimensions and/or volume and/or morphology of the healthy bony tissue and or of erosions are obtained as regards dimensions and/or volume and/or morphology of the healthy bony tissue and/or erosions of one or more known clinical cases, and so a numerical indication about the presence/absence of a bony damage and/or an indication about the evolution condition of said bony damage i.e. a staging of the bony damage.

As regards shapes it is to be noted that there is the possibility of defining a set of basic primitive geometric shapes that can be associated each one to a numerical value, further defining parameters indicating the type and the entity of a change from the primitive shape due to a deformation and there being possible to define numerical values of the combination of said primitive shapes. Therefore to each shape it is possible to associate a numerical parameter or a combination of numerical parameters defining the shape in an objective way and numerically coding it.

The ratio between volume of erosions and/or volume of healthy tissue is determined by defining the erosion volume as the difference of dimensions and shape of bony tissue as determined by images of the body subjcted to examination with typical dimensions and typical shape for example average dimensions and average shape of the bony tissue, the volume of the healthy bony tissue being determined by the difference of the erosion volume determined in this way and the average volume or the volume typical for the bony tissue without diseases, data relevant to dimensions, volume and morphology of the bony tissue without diseases being determined by the database of known clinical cases.

According to a variant the set of pixels or voxels or image data reproducing the image of the bony tissue in the MRI image can also be compared with the image of the same bony tissue obtained by dimension and/or volume and/or morphologic data of the database of known clinical cases, a morphing or registering process being carried out for bring said images of the bony tissue under congruence and which process gives numerical indications of dimension, volume and morphologic differences between the bony tissue represented in the image of the body subjected to examination and the bony tissue having typical dimensions, and/or volume and/or shape and relevant to the database of clinical cases. Therefore these numerical indications refer to deformation necessary for bringing images of the bony tissue under registration and congruence and indicate a registration and/or congruence error constuting a measure of the bony damage. Typical values of dimensions, volume and morphology of bony tissue without rheumatic disease with different evolution conditions of a rheumatic disease being established, by the comparison of typical values with corresponding values determined by the subsection determining the bony damage being determined the presence or absence of the disease and/or the evolution degree of the disease or by the morphing and/or registering process of images of the bony tissue corresponding to said typical dimension, volume and morphology values being established the presence/absence of the disease and/or the evolution degree thereof.

Each one of these processing tools provides one or more numerical parameters just describing some quantities that are considered to be valid for revealing the presence of the rheumatic disease and/or for determining the evolution condition of the rheumatic disease. They are indicated by P1, P2, P3, P4, P5, P6. It is also possible to combine such parameters with one or more further parameters P7 that have been determined with other kinds of examinations and/or that are about the patient history or personal data and/or pathologic conditions according to previous examinations.

Parameters P1 to P7 are not all necessary and some of them can be omitted or they can be considered as to have a different importance and therefore are evaluated in a different way one with respect to the other.

A mode for determining the presence of the rheumatic disease and/or the evolution stage of such disease according to a very simplified embodiment is the simple comparison for said parameters with threshold numerical values.

In this case each parameter can be individually evaluated or it is possible to consider said parameters as being part of a vector of pahtologic conditions and so to provide an overall evaluation of measured parameters and of threshold ones in the form of a comparison of the length of the corresponding vector whose components are composed of measured parameters P1 to P7 for one of the vectors and of threshold values for said parameters for the comparison one.

Again a variant provides individual parameters to be weighted when their importance is different for determining the diagnostic indication.

For determining the presence of the rheumatic disease and/or the evolution condition thereof it is also possible to use other statistic systems evaluating parameters P1 to P7 or any subcombination thereof.

Finally a particularly developped embodiment provides numerical parameters P1 to P7 to be input values of a classification algorithm or a predictive algorithm such as for example an artificial neural network indicated by 119 in figure 3.

In this case the database of clinical cases and particularly of the specific patient are used for carrying out the training and testing step of the neural network.

It is interesting to consider the fact that the general database can lack in all or some specific data of the patient and so the network can be in an intermediate learning condition, while the learning ends during the step examining the patient by loading the personal clinical database of the patient and by carrying out a further training and testing step by this database. Clinical data of the patient relevant to other preceding examinations both of the same type and of the different type can be stored in a storing movable medium such as a chip-card also known as smart card or the like that is read by the system at the imminence of an examination session by a suitable reader.

Therefore the system according to the present invention have to allow the storing of data obtained by above described methods during different examinations made on each patient.

The personal clinical database of the patient allows also to evaluate in a precise, economic, and rapid way the evolution of the disease both with a theraphy and without it by means of a follow-up both of the simple patologic condition and possibly or as an alternative of the therapeutic treatment of the rheumatic disease.

Also in this case, the system provides a processing module or tool working on the basis of automatic comparison algorithms that for example are based on "expert systems" or predective ones and intended to highlight changes occurred between an examination and the other one during the therapeutic treatment.

From the above it is clear that the system according to the present invention provides methods for measuring function parameters intended to highlight the condition of the rheumatic disease and to allow its follow-up during the theraphy and specifically these methods consist in means for processing images for analysing the synovial condition by highlighting the inflammatory condition, such as the measurement of characteristic parameters of the paramagnetic contrast medium perfusion in the synovia, and/or the comparative analysis of RM image contrast obtained at different stages of the disease. As regards the cartilage the invention provides the segmentation and that is the measure as regards thicknesses or overall volume intended to highlight the patologic condition and the evolution during the theraphy, as well as the analysis regarding RM contrast aiming at highlighting pathologic regions in the more automatic possible way. At last the invention provides also means for staging the bony damage automatically or semi-automatically highlithing the amount of bony erosions and their volume with respect to the volume of healthy bone.

It is also important to consider the fact that the system according to the present invention allows to deduce diagnosis suppositions or however to provide the medical staff with widening/highlighting cues and not with an automatic definitive diagnosis by the comparison with quantitative parameters measured during the occuring examination.

With a particular reference to the making of means for acquiring MRI images, according to a preferrd embodiment of the invention they are composed of a scanner with a magnetic structure having permanent magnets, preferably made of neodymium. The field direction is provided as transversal to the magnet axis. The magnetic field is provided to have the following characteristics:
Static field intensity: 0.21 T ± .7 mT (f0: 8.93 MHz ± 300 KHz)
Homogeneity: < ± 4 ppm FWHM on 140 mm DSV
Shimming system: passive
Possibility of modifying the magnetic field: ±1 mT (± 45 KHz).
Dispesed field (line at 0.5 mT): within 1 meter from magnetic unit
Magnetic shielding: not necessary

Other parameters are the following:
Gantry opening: usable width at least 18 cm; height to be defined
Space resolution: up to 0.4 mm
View: 140 mm x 140 mm

Advantageously there is provided the use of pole pieces for a considerable Eddy current reduction. Receiving coils with Dual Phased Array technology.

Gradient system provides a maximum intensity: included between ± 15 mT/m and ± 20 mT/m a rise time: 0.5 ms from 0 to +15 mT/m at 99% and a Linearity : ± 5 % on 140 mm DSV.

In combination there are also provided RF electromagnetic shielding means.

As regards electronic/information characteristic of the tomograph there is provided:
- Operating system working in Windows environment, implemented on PC hardware;
- Native implementation of DICOM protocol;
- Software application "operator interface" uniform for standard PC working station of the tomograph and for remote PC working station allowing to carry out tele-diagnosis;
- Software application for transmitting texts and image in real time, even during the examination;
- Devices intended to allow automatic adjustments and calibrations, also remote ones (teleervice).
As regards functional-applicative characteristics:
- DPA coils specific for examining joints, at least knee and hand/wrist, complete with supports for a confortable positioning and a precise optmization of signal-noise ratio;
- Auxiliary devices for examining kinematics of joints;
- Real-time displaying for make the limb positioning easier;
- Standard set of MRI sequences enlarged by:
   - Fast Spin Echo sequences (FSE) for optimizing weighted images T2;
   - Sequences allowing the suppression of fat signal (fat suppression).
      low field, of the Fast Spin Echo STIR and DIXON type
   - set of 3D acquisition sequences with different contrast (T1, T2) both in echo gradient and spin-echo, aiming at developping methods for the 3D segmentation of organs under interest
   - method acquiring arrays up to 512x512 for high resolution
   - package for analysing the spreading rate of the contrast medium composed of
      acquisition sequences and a software for analysing and showing data
   - set of SE sequences for detecting maps in T1 and T2.

## Claims

1. Apparatus for determining indications helping the diagnosis of rheumatic diseases comprising at least a section for detecting and acquiring signals from a body under examination or a part thereof
**characterized in that**
the detection section is a unit for detecting images by nuclear magnetic resonance;
and in said apparaus there is further integrated a section for processing acquired images as regards image data and/or resonance signals, which processing section from image data and/or resonance signals, determines values of one or more different numerical parameters indicating the presence or absence of a rheumatic disease and/or a measure of the evolution condition of said rheumatic disease.

2. Apparatus according to claim 1, **characterized in that** the section for acquiring images by nuclear magnetic resonance has scanning means with such a shape and dimension to acquire nuclear magnetic resonance images only for some limited parts of a body under examination and/or for some limited anatomical regions or even for some limited parts of said anatomical regions.

3. Apparatus according to claims 1 or 2, **characterized in that** MRI scanning means have such a shape and dimension that allow the acquisition of MRI images from anatomical regions of one or more joints like hand, wrist, and knee there being provided a first configuration with shape and dimension intended to allow the acquisition of images of the anatomical region of knee, ankle, foot, elbow, hand and wrist and possibly also of the shoulder when the magnetic structure is of the open type or having a C or U-shaped section and/or there being provided a second configuration with such shape and dimension to allow the acquisition of images only for anatomical regions of the hand, wrist, ankle and foot and possibly a third alternative configuration for acquiring images only of the hand and fingers.

4. Apparatus according to one or more of the preceding claims, **characterized in that** the processing section comprises a sub-section for determining the synovial inflammatory condition.

5. Apparatus according to one or more of the preceding claims, **characterized in that** the processing section comprises a sub-section for determining dimensions and/or the morphology of the cartilage in the anatomical region under examination and particularly of the hand and/or knee.

6. Apparatus according to one or more of the preceding claims, **characterized in that** it comprises a subsection for determining conditions of the cartilage tissue by means of an analysis by detecting contrast maps of the T1 and/or T2 type and/or by means of the image analysis by contrast media.

7. Apparatus according to one or more of the preceding claims, **characterized in that** the processing section comprises a sub-section for determining the presence and/or the condition of damages to the bony tissue.

8. Apparatus according to one or more of the preceding claims, **characterized in that** the processing section is composed of a computer wherein a program managing the processing of image data and/or resonance signals is loaded or can be loaded and is executed or can be executed therefrom, whereas each one of the processing sub-sections is composed of a specific processing program that is loaded or can be loaded by it, which processing program is executed by the call-up and/or under the control of the program managing the processing.

9. Apparatus according to one or more of the preceding claims, **characterized in that** the sub-section determining the synovial inflammatory condition comprises means for determining perfusion parameters of the paramagnetic contrast medium in the synovia and means for comparing said parameters with an reference value for discriminating the presence/absence of a synovial inflammatory condition and/or for the comparison with a reference scale for determining a parameter indicating the evolution degree of the synovial inflammatory condition, which reference value for discriminating the presence/absence of the synovial inflammatory condition and/or which reference scale for determining the evolution degree of the inflammatory condition are included in a database of known clinical cases.

10. Apparatus according to one or more of the preceding claims, **characterized in that** the sub-section determining the synovial inflammatory condition comprises means for determining numerical values of said synovial inflammatory condition both regarding the presence/absence of it and the evolution condition of the inflammation by means of the comparative analysis of the contrast of RM images such as the detection of maps in T1 or T2, obtained at different stages of the inflammatory condition from images relevant to patients being part of the database of known clinical cases and/or from images relevant to previous indagations carried out on the same patient.

11. Apparatus according to one or more of the preceding claims, **characterized in that** the sub-section sub-section determining the synovial inflammatory condition comprises means for determining numerical values of said synovial inflammatory condition both regarding the presence/absence of it and the evolution condition of the inflammation by processing image data by classification algorithms and/or predictive algorithms, which have been trained (training and testing) by image data relevant to the synovia of known clinical cases included in a database of known clinical cases.

12. Apparatus according to one or more of the preceding claims, **characterized in that** it provides a sub-section segmenting image data for recognizing and delimiting one or more image regions comprising subsets of pixels or voxels, measurements of parameters or of perfusion curves being carried out within one or more of said limited image regions.

13. Apparatus according to one or more of the preceding claims, **characterized in that** the sub-section determining dimensions and/or the morphology of the cartilage, in an anatomical region, particulraly of the hand and/or knee, comprises means segmenting images for determining subsets of image data and/or pixels and/or voxels and for identifying real objects that are reppresented in the image by said subsets of image data and/or pixels and/or voxels, the subset of pixels or voxels or image data reppresenting the cartilage being identified and dimensions and/or the shape of said cartilage being determined and/or that sub-section further comprises means for analysing the condition of the cartilage tissue by contrast maps T1 and/or T2 or analysing the image by contrast media.

14. Apparatus according to claim 13, **characterized in that** the sub-system determining the dimensions and/or the morphology of the cartilage in an anatomical region and particularly of the hand and/or knee further comprises means for generating a virtual image reconstructed on the basis of image data processed by segmentation means, such as rendering means possibly combined with morphing and/or smoothing means.

15. Apparatus according to one or more of claims 12 to 14, **characterized in that** the sub-section determining dimensions and/or the morphology of the cartilage and/or the tissue condition in an anatomical region and particularly of the hand and/or knee comprises means for comparing dimension and/or morphologic data and/or data about the tissue condition of the cartilage with dimension and morphologic data and/or data about the tissue condition of a plurality of know clinical cases that are kept in a database of clinical cases, which comparison provides information about the differences and indentities of dimensions and/or morphology and tissue condition of the cartilage with respect to dimensions and/or morphology and/or tissue condition of the cartilage of one or more known clinical cases, and therefore it provides an indication about the presence /absence of a rheumatic disease and/or an indication about the evolution condition of said rheumatic disease.

16. Apparatus according to one or more of claims 12 to 15, **characterized in that** from a database of known clinical cases comprising images in an anatomical region and particularly of the hand and/or knee dimension and/or morphologic data of the csrtilage are determined by means for determining the dimensions and/or the morphology and/or the tissue condition of the cartilage in said anatomical region working by a segmentation and/or rendering and/or morphing and/or smoothing processing of image data at least a reference value being determined for discriminating the presence/absence of a rheumatic disease and/or a scale and/or a function correlating different dimension and/or morphologic conditions and/or tissue condition of the cartilage and different evolution degrees of the disease, whereas dimension and morphologic values and/or tissue condition values of the cartilage for a case under examination determined by the corresponding subset are compared with said reference value and with said scale or said correlation function for determining an indication about the presence/absence and/or the evolution degree of a rheumatic disease.

17. Apparatus according to one or more of the preceding claims, **characterized in that** the sub-system or sub-section determining the presence and/or the condition of damages to the bony tissue, particularly in the anatomical region of the hand and/or of the knee, comprises means for automatically or semi-automatically determining the amount of bony erosions and the volume thereof, as well as the ratio between the volume of bony erosions with respect to the volume of healthy bone.

18. Apparatus according to claim 17, **characterized in that** means for automatically or semiautomatically determining the amount of bony erosions and the volume thereof, as well as the ratio between the volume of bony erosions with respect to the volume of healthy bone are composed of means segmenting images for determining subsets of image data and/or pixels and/or voxels and for identifying real objects that are shown in the image from said subsets of image data and/or pixels and/or voxels, the subset of pixels or voxels or image data reppresenting the bony tissue being identified.

19. Apparatus according to claim 17 or 18, **characterized in that** the sub-system determining the presence and/or the condition of damages to the bony tissue, in an anatomical region and particularly of the hand and/or of the knee, further comprises means for generating a virtual image reconstructed on the basis of image data processed by segmentation means, such as rendering means possibly combined with morphing and/or smoothing means.

20. Apparatus according to one or more of claims 17 to 19, **characterized in that** the sub-section determining the presence and/or the condition of bony tissue damages, in an anatomical region and particularly in the anatomical region of the hand and/or knee comprises means determining dimensions and/or the morphology of the bony tissue from subsets of pixels and/or voxels and/or image data that have been identified as representing the bony tissue in the body part under indagation, whereas erosions, dimensions and the morphology thereof are determined by comparing dimension and/or morphologic data of the bony tissue obtained by processing the MRI image or images of the body subjected to indagation with dimension and/or morphologic data of a plurality of known clinical cases kept in a database of clinical cases, which comparison provides information about differences and identitites of dimensions and/or morphology of the healthy bony tissue and/or of erosions with respect to dimensions and/or the morphology of the healthy bony tissue and/or erosions of one or more known clinical cases, and therefore provides an indication about the presence /absence of a bony damage and/or an indication about the evolution condition of said bony damage i.e. a staging of the bony damage.

21. Apparatus according to one or more of claims 17 to 20, **characterized in that** the ratio between the volume of erosions and/or the volume of healthy bony tissue is determined by determining the volume of erosions by the difference of dimensions and of the shape of the bony tissue as determined by images of the body under indagation with typical dimensions and the typical shape for example average dimensions and average conformation of the bony tissue, the volume of healthy bony tissue being determined by the difference of the erosion volume determined in such way and the average volume or typical volume for the bony tissue without a disease, data relevant to dimensions, volume and morphology of the bony tissue without a disease being determined by means of the database of known clinical cases.

22. Apparatus according to one or more of claims, **characterized in that** the set of pixels or voxels or image data reproducing the image of the bony tissue in the MRI image is compared with the image of the same bony tissue obtained from dimension and/or volume and/or morphologic data of the database of known clinical cases, a morphing or registering process being carried out in order to bring said images of the bony tissue under congruence and which process provides numerical information about dimension, volume and morphologic differences between the bony tissue reppresented in the image of the body under indagation and the bony tissue having typical dimensions and/or volume and/or shape and relevant to the database of clinical cases, which numerical indications refer to deformations necessary to bring images of the bony tissue under registration and congruence and indicate a registration and/or congruence error constituting a measure of the bony damage.

23. Apparatus according to one or more of claims 17 to 22, **characterized in that** typical values of dimensions, volume and morphology of the bony tissue without a rheumatic disease with different evolution conditions of a rheumatic disease are set, the presence or absence of a disease and/or the evolution degree of the disease being determined by the comparison between typical values and corresponding values determined by the sub-section determining the bony damage or the presence/absence of the disease and/or the evolution degree thereof being determined by the morphing and/or registering process of images of the bony tissue corresponding to said typical dimension volume and morphologic values.

24. Apparatus according to one or more of the preceding claims, **characterized in that** it comprises means for verifying the reliability in identifying the subset of pixels or voxels or image data reppresenting in the image or in the set of image data a real object and particularly the cartilage and/or the bony tissue and/or restricted regions for evaluating the perfusion, which verifying means comprise a database of dimension and/or volume and/or morphologic configuration data, i.e. of typical shapes of the cartilage and/or of the bony tissue and/or regions evaluating the perfusion under specific conditions of absence or presence of a rheumatic disease and/or a specific evolution degree of the rheumatic disease, whereas dimensions and/or volume and/or the morphology of the cartilage, of the bony tissue and/or of perfusion evaluating regions determined by sub-sections of processing means are compared with said typical dimension and/or volume data and/or with typical morphologies for the cartilage, for the bony tissue and for perfusion evaluating regions that are in the form of average values and/or a range of average values of dimension and/or volume and/or morphologic differences, a first maximum difference threshold being set, whose exceeding makes dimension and/or volume and/or morphologic data provided by sub-sections to be considered as unreliable and incompatible ones, verifying means being provided with a sub-section signalling or requiring the repetition of the process for determining dimensions and/or volume and/or morphologic characteritics and/or of the acquisition of MRI image or images or said verifying means are provided with a sub-section automatically commanding the repetition of the process for determining dimensions and/or volume and/or morphologic characteristics and/or the acquisition of MRI image or images.

25. Apparatus according to one or more of the preceding claims, **characterized in that** the detecting section, particularly the unit detecting images by nuclear magnetic resonance is connected to the section processing acquired images by means of a feed-back line.

26. Apparatus according to claim 25, **characterized in that** the processing section controls the change of parameters setting the detecting section and/or the choice or the change of image acquisition sequences carried out by the detection section for acquiring images on the basis of a verification of the quality of image data and/or of resonance signals with reference to characteristics of image data and/or resonance signals important for carrying out the processing processes carried out by the processing section

27. Apparatus according to claims 25 or 26, **characterized in that** the detection section by the feed-back line controls the processing section in treating image data and/or resonance signals by one or more different sub-sections and according to a predetermined order on the basis of the greatest quality that can be obtained from image data and/or signals and/or the length of the acquiring process, of the length of the processing process and of the reliability of results of the image processing expressed in the form of statistic reliability or error parameters and/or in the form of fitness.

28. Apparatus according to one or more of the preceding claims 25 to 27, **characterized in that** it comprises a unit generating setting parameters of the detection section, particularly of means acquiring MRI images and/or selecting and/or generating and/or setting acqusition sequences, which unit can be controlled by a unit verifying the quality of image data and/or of resonance signals and/or evaluating statistic reliability or error parameters and/or in the form of fitness and which verification unit controls the unit generating setting parameters of the detection section, particularly of means acquiring MRI images and/or selecting and/or generating and/or setting acquisition sequences upon the generation of new setting parameters and/or choice and/or generation and/or setting of new acquisition sequences on the basis of results of the quality verification of image data and/or resonance signals and/or evaluation of statistic reliability or error parameters and/or in the form of fitness.

29. Apparatus according to claim 28, **characterized in that** the verification unit works in an iterative way and in this case it is provided with a counter for setting a maximum amount of activations for the control generating setting parameters of the detecting section, particularly of means acquiring MRI images and/or selecting and/or generating and/or shaping acquisition sequences and/or of means setting a minimum quality threshold of image data and/or of resonance signals and/or of error or statistic reliability and/or fitness of processing results.

30. Apparatus according to one or more of the preceding claims, **characterized in that** the processing section provides a sub-section for the recapitulatory definition of the indication about the probable presence or absence of a rheumatic disease and/or the probable evolution condition of the diases comprising classification and/or predictive means such as for example a classification algorithm and/or a predictive algorithm that are trained by data of a database of known clinical cases and to which there are provided parameters indicating the presence/absence of a disease and/or the evolution degree of the disease as input data determined by sub-sections determining the synovial inflammatory condition, determining dimensions and/or morphology of the cartilage in the anatomical region of the hand and/or knee, determining the presence and/or the state of damages to the bony tissue and possibly further data obtained by different examination and/or personal data or the medical history of the patient.

31. Apparatus according to one or more of the preceding claims, **characterized in that** each patient is provided with a dedicated personal medium for storing diagnostic data of previous examinations relevant to a rheumatic disease and possibly also to other diseases, said data being used for the comparison with data obtained by subsequent examinations and the definition of the disease evolution and of the therapy efficacy, the apparatus being provided with readers for said storing personal medium.

32. Method for determining indications helping the diagnosis of rheumatic diseases comprising steps of
acquiring one or more images by nuclear magnetic resonance and subjecting image data and/or resonance signals to a processing for determining from image data and/or resonance signals, values of one or more numerical parameters indicating of the presence or absence of a rheumatic disease and/or a measure of the evolution condition of said rheumatic disease, said processing being carried out immediately after the acquisition of the image or images under nuclear magnetic resonance.

33. Method according to claim 32, **characterized in that** it provides the acquisition of MRI images from anatomical regions of the hand and knee.

34. Method according to claims 32 or 33, **characterized in that** the presence or absence and/or the evolution degree of the rheumatic disease is determined as an alternative or in combination by determining from one or more MRI images and/or from resonance signals numerical parameters describing the synovial inflammatory condition and/or by determining from one or more MRI images and/or from magnetic resonance signals dimensions and/or the morphology and/or the condition of the cartilage condition in an anatomical region of a joint and particularly in the anatomical region of the hand and/or knee and/or by determining from MRI images and/or from magnetic resonance signals the damage presence and degree, particularly of erosions to the bony tissue.

35. Method according to claim 34, **characterized in that** the synovial inflammatory condition is measured by determining perfusion curves of a paramagnetic contrast medium and by comparing them with perfusion curves relevant to known clinical cases included in a adatabase of known clinical cases.

36. Method according to claims 34 or 35, **characterized in that** the synovial inflammatory condition is measured by comparing detected image contrast maps with image contrast maps relevant known clinical cases.

37. Method according to one or more of the preceding claims 34 to 36, **characterized in that** dimensions and/or morphology of the cartilage in the anatomical region of the hand and/or knee are determined by segmenting the acquired MRI image or images and by identifying subsets of pixels or voxels or image data representing the cartilage in acquired MRI images and by measuring dimensions and shape characteristics of the cartlage as a function of dimensions and shape characteristics of the subset of pixels or voxels or image data representing it in the image.

38. Method according to claim 37, **characterized in that** the image or images and/or image data are further subjected to a rendering process and possibly to a morphing and/or smoothing process before determining dimensions and/or shape characteristics of subsets of pixels or voxels or image data representing the cartilage.

39. Method according to claims 37 or 38, **characterized in that** a numerical parameter about the presence or absence of the rheumatic disease and/or for evaluating the evolution degree of the rheumatic disease is set according to a reference scale by comparing dimensions and/or shape characteristics of subsets of pixels or voxels or image data representing the cartilage in acquired images with reference values and/or with a reference scale determined by dimension data and/or shape characteristics data of the cartilage in a plurality of known cases being part of a database of known clinical cases.

40. Method according to claim 39, **characterized in that** it provides a step for verifying the reliability in identifying as the representation of the cartilage in acquired images of the subset of pixels or voxels or image data obtained by the segmentation process and/or possible further rendering process possibly also in combination with a morphing and/or smoothing process, in which verification step dimension and shape characteristics determined as a function of said subset of pixels or voxels or image data are compared with typical average values or with typical morphologic characteristics or with ranges of said values and of said morphologic characteristics, a maximum difference threshold being provided, so when the result of the comparison is greater than said difference threshold the definition of the subset of pixels or voxels or image data and/or the acquired image or images are considered as wrong ones and a new segmentation process and/or a possible further rendering process possibly also in combination with a morphing and/or smoothing process or a new MRI image acquisition is required or is automatically carried out.

41. Method according to one or more of the preceding claims 34 to 40 **characterized in that** the presence and/or the condition of damages to the bony tissue, particularly in the anatomical region of the hand and/or knee are automatically or semi-automatically determined on the basis of the amount of bony erosions and to the volume thereof, as well as on the basis of the ratio between the volume of bony erosions with respect to the volume of healthy bone.

42. Method according to claim 41, **characterized in that** means for automatically or semi-automatically determining the amount of bony erosions and the volume thereof, as well as the ratio between the volume of bony erosions with respect to the volume of healthy bone provides steps for segmenting images for determining subsets of image data and/or pixels and/or voxels and for identifying real objects that are represented in the image by said subsets of image data and/or pixels and/or voxels, the subset of pixels or voxels or image data representing the bony tissue being identified.

43. Method according to claim 41, **characterized in that** it further provides the step for generating a virtual image reconstructed on the basis of image data processed in the segmentation process, i.e. carrying out a rendering process possibly combined with a morphing and/or smoothing process on the basis of image data subjected to segmentation.

44. Method according to one or more of claims 41 to 43, **characterized in that** it provides steps for determining the presence and/or the condition of bony tissue damages, particularly in the anatomical region of the hand and/or knee by determining dimensions and/or volume and/or the morphology of the bony tissue as a function of dimensions and/or volume and/or morphology from subsets of pixels and/or voxels and/or image data that have been identified as representing the bony tissue in the body part under indagation, whereas erosions, dimensions and the morphology thereof are determined by comparing dimension and/or volume and/or morphologic data of the bony tissue obtained by processing the MRI image or images of the body subjected to indagation with dimension and/or volume and/or morphologic data of a plurality of known clinical cases kept in a database of clinical cases, which comparison provides information about differences and identitites of dimension and/or volume and/or morphology of the healthy bony tissue and/or of erosions as regards dimensions and/or volume and/or the morphology of the healthy bony tissue and/or of erosions of one or more known clinical cases, and therefore provides an indication about the presence /absence of a bony damage and/or an indication about the evolution condition of said bony damage i.e. a staging of the bony damage.

45. Method according to one or more of claims 41 to 44, **characterized in that** the ratio between the volume of erosions and/or the volume of healthy bony tissue is determined by determining the volume of erosions by the difference of dimensions and of the shape of the bony tissue as determined by images of the body under indagation with typical dimensions and the typical shape for example average dimensions and average conformation of the bony tissue, the volume of healthy bony tissue being determined by the difference of the erosion volume determined in such way and the average volume or typical volume for the bony tissue without a disease, data relevant to dimensions, volume and morphology of the bony tissue without a disease being determined by means of the database of known clinical cases.

46. Method according to claim 45, **characterized in that** the set of pixels or voxels or image data reproducing the image of the bony tissue in the MRI image is compared with the image of the same bony tissue obtained from dimension and/or volume and/or morphologic data of the database of known clinical cases, a morphing or registering process being carried out in order to bring said images of the bony tissue under congruence and which process provides numerical information about dimension, volume and morphologic differences between the bony tissue reppresented in the image of the body under indagation and the bony tissue having typical dimensions and/or volume and/or shape and relevant to the database of clinical cases, which numerical indications refer to deformations necessary to bring images of the bony tissue under registration and congruence and indicate a registration or congruence error constituting a measure of the bony damage.

47. Method according to one or more of claims 41 to 46, **characterized in that** typical values of dimensions, volume and morphology of the bony tissue without a rheumatic disease with different evolution conditions of a rheumatic disease are set, the presence or absence of a disease and/or the evolution degree of the disease being determined by the comparison between typical values and corresponding values determined by the sub-section determining the bony damage or the presence/absence of the disease and/or the evolution degree thereof being determined by the morphing and/or registering process of images of the bony tissue corresponding to said typical dimension volume and morphologic values.

48. Method according to one or more of the preceding claims 32 to 47, **characterized in that** there is provided a feed-back interaction between the detecting section, particularly the unit for detecting images by nuclear magnetic resonance the section processing acquired images which feed-back interaction provides for the processing section to control the change of setting parameters of the detecting section and/or the choice or change of image acquisition sequences carried out by the detecting section for acquiring images on the basis of the result of a verification of the quality of image data and/or resonance signals with reference to characteristics of image data and/or resonance signals important for carrying out processing processes carried out by the processing section.

49. Method according to claim 48, **characterized in that** it provides to change setting parameters of the detecting section and/or the choice or change of image acquisition sequences as a function of the maximum quality that can be obtained of image data and/or signals and/or length of the acquisition process.

50. Method according to claim 49, **characterized in that** it provides to change image processing steps with regards to the tecnique for treating image data and/or to the carrying out order as a function of the length of the processing process and of the reliability of results of the image processing expressed in the form of statistic reliability or error parameters and/or in the form of fitness.

51. Method according to claims 49 or 50, **characterized in that** it provides to iteratively carry out steps for verifying the maximum quality that can be obtained of image data and/or signals and/or length of the acquisition process and/or also of the length of the processing process and of the reliability of results of image processing expressed in the form of statistic reliability or error parameters and/or in the form of fitness and steps modifying setting parameters of the detecting section and/or the choice or change of image acquisition sequences and/or
